# EUROPEAN PATENT APPLICATION

(11) **EP 4 311 277 A1**
(43) Date of publication of application: **24.01.2024**
(21) Application number: 22794452.7
(22) Date of filing: 28.03.2022
(51) Int. Cl.: H04W 4/029, H04W 4/80, H04W 12/02, G16H 50/80

(54) **CONTACT TRACING METHOD AND RELATED DEVICE**

(30) Priority: 30.04.2021 CN 202110487648
(71) Applicant: Huawei Technologies Co., Ltd., Shenzhen, Guangdong 518129 (CN)
(72) Inventor: QIN, Peng, Shenzhen, Guangdong 518129 (CN); ZOU, Tao, Shenzhen, Guangdong 518129 (CN); LI, Tong, Shenzhen, Guangdong 518129 (CN); YAN, Lu, Shenzhen, Guangdong 518129 (CN)
(74) Representative: MERH-IP Matias Erny Reichl Hoffmann Patentanwälte PartG mbB
(86) International application number: PCT/CN2022/083393
(87) International publication number: WO 2022/227979

(57) **Abstract**

Embodiments of this application disclose a contact tracing method. An electronic device may upload a periodic key of a user to a server, and periodically derive a working key based on the periodic key. The electronic device generates a Bluetooth advertising packet based on the working key. Bluetooth advertising packets are sent and received between different electronic devices. When a target user appears, the server searches for a periodic key of the target user, derives all working keys based on the periodic key to obtain a working key set of the target user, and sends the working key set to electronic devices of all users. The electronic device performs contact tracing and prediction on a suspected target user based on the working key set of the target user and a previously received Bluetooth advertising packet. This resolves a problem that uploaded key information is incomplete or inaccurate. In addition, after receiving contact information uploaded by the suspected target user, the server may generate a contact chain based on the contact information, to assist a related staff member in performing source tracing for an infection source of the target user.

## Description

This application claims priority to Chinese Patent Application No. 2021104876486, filed with the China National Intellectual Property Administration on April 30, 2021 and entitled "CONTACT TRACING METHOD AND RELATED DEVICE", which is incorporated herein by reference in its entirety.

### TECHNICAL FIELD

This application relates to the field of communication technologies, and in particular, to a contact tracing method and a related device.

### BACKGROUND

At present, many application scenarios relate to efficient tracing for a contact person. For example, in a process of preventing and controlling some infectious diseases, a person who has a history of contact with a patient of an infectious disease needs to be traced in a timely manner. In a process of tracing a contact person, a server usually needs to use some applications to obtain user information. However, a problem that uploaded user information is incomplete or inaccurate often occurs in these applications, and efficient tracing cannot be implemented.

### SUMMARY

Embodiments of this application provide a contact tracing method, to efficiently trace a contact user of a target user, and resolve a problem that uploaded user information is incomplete or inaccurate.

According to a first aspect, an embodiment of this application provides a contact tracing method, applied to a communication system including a first electronic device, a second electronic device, and a first server. The method includes: The first electronic device establishes a short-range wireless communication connection to the second electronic device; the first electronic device generates a first periodic key, and sends the first periodic key to the first server; the first electronic device sends a first working key to the second electronic device through the short-range wireless communication connection, where the first working key is derived by the first electronic device based on the first periodic key; the second electronic device receives and stores the first working key; the first server determines that a user of the first electronic device is a target user; the first server derives a first working key set based on the first periodic key, and sends the first working key set to the second electronic device; and if the second electronic device obtains the first working key set and determines that the first working key set includes the first working key, the second electronic device determines that a user of the second electronic device is a suspected target user.

In this embodiment of this application, according to the method provided in the first aspect, an electronic device may upload a periodic key of a user to a server for storage, and periodically derive a working key based on the periodic key. When the electronic device enables Bluetooth low energy, the electronic device may generate a Bluetooth low energy advertising packet based on the working key. Bluetooth low energy advertising packets may be sent and received between different electronic devices. When a target user appears, the server may search for a periodic key of the target user, derive all working keys based on the periodic key to obtain a working key set of the target user, and send the working key set to electronic devices of all users. The electronic device may perform contact tracing and prediction on a suspected target user based on the working key set of the target user and a previously received Bluetooth low energy advertising packet. This resolves a problem that uploaded user information is incomplete or inaccurate. In addition, after receiving contact information uploaded by the suspected target user, the server may generate a contact chain based on the contact information, to assist a related staff member in performing source tracing for an infection source of the target user.

In a possible implementation, the first periodic key is periodically generated by the first electronic device and sent to the first server. In this way, user privacy security can be protected.

In a possible implementation, before the first electronic device sends a first working key to the second electronic device through the short-range wireless communication connection, the method further includes: The first electronic device sends first user information to the first server, where the first user information is associated with the first periodic key; and the second electronic device sends second user information to the first server. In this way, the server can store user information and associate the user information with a periodic key.

In a possible implementation, after the first electronic device establishes a short-range wireless communication connection to the second electronic device, the method further includes: The second electronic device sends a second working key to the first electronic device, where the second working key is derived by the second electronic device based on a second periodic key by using a first derivation algorithm, the second periodic key is associated with the second user information, and the second periodic key is periodically generated by the second electronic device and sent to the first server; and the first electronic device receives and stores the second working key. In this way, working keys can be sent and received between electronic devices.

In a possible implementation, the first working key is derived by the first electronic device from the first periodic key by using the first derivation algorithm, and the first working key set is derived by the first server from the first periodic key by using the first derivation algorithm. In this way, it can be ensured that an electronic device and a server use a same derivation algorithm.

In a possible implementation, after the second electronic device determines that a user of the second electronic device is a suspected target user, the method further includes: The second electronic device sends first contact information and the second user information to the first server, where the first contact information includes the first working key; and the first server generates a first contact chain based on the first contact information and the second user information, where the first contact chain indicates that the user of the first electronic device was in contact with the user of the second electronic device. In this way, a contact chain can be used to assist a staff member in performing source tracing for an infection source of the target user.

In a possible implementation, before the second electronic device sends first contact information and the second user information to the first server, the method further includes: The second electronic device outputs first prompt information, where the first prompt information indicates that the user of the second electronic device is the suspected target user; and the second electronic device detects a first operation of the user. In this way, the user may be notified whether the user is a suspected target user, and if the user is a suspected target user, the user may be prompted to upload contact information.

In a possible implementation, the communication system further includes a third electronic device, and before the first server generates a first contact chain based on the first contact information and the second user information, the method further includes: The third electronic device sends third user information to the first server, where the third user information is associated with a third periodic key, and the third periodic key is periodically generated by the third electronic device and sent to the first server; the third electronic device establishes a short-range wireless communication connection to the second electronic device; the third electronic device sends a third working key to the second electronic device through the short-range wireless communication connection, where the third working key is derived by the third electronic device based on the third periodic key; the second electronic device receives and stores the third working key; the second electronic device sends the second working key to the third electronic device through the short-range wireless communication connection; and the third electronic device receives and stores the second working key. In this way, working keys can be sent and received between electronic devices.

In a possible implementation, after the first server generates a first contact chain based on the first contact information and the second user information, the method further includes: The first server obtains the second periodic key based on the second user information; the first server derives a second working key set based on the second periodic key, and sends the second working key set to the third electronic device; and if the third electronic device obtains the second working key set and determines that the second working key set includes the second working key, the third electronic device determines that a user of the third electronic device is a contact user of the suspected target user. In this way, whether a user is a contact user of the suspected target user can be determined.

In a possible implementation, after the third electronic device determines that a user of the third electronic device is a contact user of the suspected target user, the method further includes: The third electronic device sends second contact information and the third user information to the first server, where the second contact information includes the second working key; and the first server generates a second contact chain based on the second contact information and the third user information, where the second contact chain indicates that the user of the third electronic device was in contact with the user of the second electronic device. In this way, contact tracing can be performed for more users by using a contact chain.

In a possible implementation, before the third electronic device sends second contact information and the third user information to the first server, the method further includes: The third electronic device outputs second prompt information, where the second prompt information indicates that the user of the third electronic device is the contact user of the suspected target user; and the third electronic device detects a second operation of the user. In this way, the user may be notified whether the user is a contact user of the suspected target user, and if the user is a contact user of the suspected target user, the user may be prompted to upload contact information.

According to a second aspect, an embodiment of this application provides a contact tracing method, applied to a communication system including a first electronic device, a second electronic device, a first server, and a second server. The method includes: The first electronic device establishes a short-range wireless communication connection to the second electronic device; the first electronic device generates a first periodic key, and sends the first periodic key to the second server; the first electronic device sends a first working key to the second electronic device through the short-range wireless communication connection, where the first working key is derived by the first electronic device based on the first periodic key; the second electronic device receives and stores the first working key; the first server sends first information to the second server; the second server determines, based on the first information, that a user of the first electronic device is a target user; the second server derives a first working key set based on the first periodic key, and sends the first working key set to the second electronic device; and if the second electronic device obtains the first working key set and determines that the first working key set includes the first working key, the second electronic device determines that a user of the second electronic device is a suspected target user. In this way, contact tracing and prediction can be performed for the suspected target user.

In a possible implementation, the first information is a set of target user information. In this way, a user does not need to actively upload diagnosis information to implement contact tracing and prediction.

In a possible implementation, the first information is confirmation information, the confirmation information indicates that the user of the first electronic device is the target user, and before the first server sends first information to the second server, the method further includes: The first electronic device sends diagnosis information to the second server; the second server sends the diagnosis information to the first server; and the first server generates the confirmation information based on the diagnosis information. In this way, a user may actively upload diagnosis information to implement contact tracing and prediction.

In a possible implementation, the first periodic key is periodically generated by the first electronic device and sent to the second server. In this way, user privacy security can be protected.

In a possible implementation, before the first electronic device sends a first working key to the second electronic device through the short-range wireless communication connection, the method further includes: The first electronic device sends first user information to the second server, where the first user information is associated with the first periodic key; and the second electronic device sends second user information to the second server. In this way, the server can store user information and associate the user information with a periodic key.

In a possible implementation, the second electronic device sends a second working key to the first electronic device, where the second working key is derived by the second electronic device based on a second periodic key by using a first derivation algorithm, the second periodic key is associated with the second user information, and the second periodic key is periodically generated by the second electronic device and sent to the second server; and the first electronic device receives and stores the second working key. In this way, working keys can be sent and received between electronic devices.

In a possible implementation, the first working key is derived by the first electronic device from the first periodic key by using the first derivation algorithm, and the first working key set is derived by the second server from the first periodic key by using the first derivation algorithm. In this way, it can be ensured that an electronic device and a server use a same derivation algorithm.

In a possible implementation, after the second electronic device determines that a user of the second electronic device is a suspected target user, the method further includes: The second electronic device sends first contact information and the second user information to the second server, where the first contact information includes the first working key; and the second server generates a first contact chain based on the first contact information and the second user information, where the first contact chain indicates that the user of the first electronic device was in contact with the user of the second electronic device. In this way, a contact chain can be used to assist a staff member in performing source tracing for an infection source of the target user.

In a possible implementation, before the second electronic device sends first contact information and the second user information to the second server, the method further includes: The second electronic device outputs first prompt information, where the first prompt information indicates that the user of the second electronic device is the suspected target user; and the second electronic device detects a first operation of the user. In this way, the user may be notified whether the user is a suspected target user, and if the user is a suspected target user, the user may be prompted to upload contact information.

In a possible implementation, the communication system further includes a third electronic device, and before the second server generates a first contact chain based on the first contact information and the second user information, the method further includes: The third electronic device sends third user information to the second server, where the third user information is associated with a third periodic key, and the third periodic key is periodically generated by the third electronic device and sent to the second server; the third electronic device establishes a short-range wireless communication connection to the second electronic device; the third electronic device sends a third working key to the second electronic device through the short-range wireless communication connection, where the third working key is derived by the third electronic device based on the third periodic key; the second electronic device receives and stores the third working key; the second electronic device sends the second working key to the third electronic device; and the third electronic device receives and stores the second working key. In this way, working keys can be sent and received between electronic devices.

In a possible implementation, after the second server generates a first contact chain based on the first contact information and the second user information, the method further includes: The second server obtains the second periodic key based on the second user information; the second server derives a second working key set based on the second periodic key by using the first derivation algorithm, and sends the second working key set to the third electronic device; and if the third electronic device obtains the second working key set and determines that the second working key set includes the second working key, the third electronic device determines that a user of the third electronic device is a contact user of the suspected target user. In this way, whether a user is a contact user of the suspected target user can be determined.

In a possible implementation, after the third electronic device determines that a user of the third electronic device is a contact user of the suspected target user, the method further includes: The third electronic device sends second contact information and the third user information to the second server, where the second contact information includes the second working key; and the second server generates a second contact chain based on the second contact information and the third user information, where the second contact chain indicates that the user of the third electronic device was in contact with the user of the second electronic device. In this way, contact tracing can be performed for more users by using a contact chain.

In a possible implementation, before the third electronic device sends second contact information and the third user information to the second server, the method further includes: The third electronic device outputs second prompt information, where the second prompt information indicates that the user of the third electronic device is the contact user of the suspected target user; and the third electronic device detects a second operation of the user. In this way, the user may be notified whether the user is a contact user of the suspected target user, and if the user is a contact user of the suspected target user, the user may be prompted to upload contact information.

According to a third aspect, an embodiment of this application provides a contact tracing method, applied to a second electronic device. The method includes: The second electronic device establishes a short-range wireless communication connection to a first electronic device; the second electronic device receives, through the short-range wireless communication connection, a first working key sent by the first electronic device, where the first working key is derived by the first electronic device based on a first periodic key, and the first periodic key is generated by the first electronic device and sent to a first server; the second electronic device receives and stores the first working key; the second electronic device receives a first working key set, where the first working key set is sent by the first server to the second electronic device after the first server determines that a user of the first electronic device is a target user, and the first working key set is derived by the first server based on the first periodic key; and if the second electronic device determines that the first working key set includes the first working key, the second electronic device determines that a user of the second electronic device is a suspected target user. In this way, contact tracing and prediction can be performed for the suspected target user.

In a possible implementation, the first periodic key is periodically generated by the first electronic device and sent to the first server. In this way, user privacy security can be protected.

In a possible implementation, the first periodic key is associated with first user information, and the first user information is sent by the first electronic device to the first server before the second electronic device receives, through the short-range wireless communication connection, the first working key sent by the first electronic device. In this way, the server can store user information and associate the user information with a periodic key.

In a possible implementation, before the second electronic device receives, through the short-range wireless communication connection, a first working key sent by the first electronic device, the method further includes: The second electronic device sends second user information to the first server. In this way, the server can store user information and associate the user information with a periodic key.

In a possible implementation, after the second electronic device establishes a short-range wireless communication connection to a first electronic device, the method further includes: The second electronic device sends a second working key to the first electronic device through the short-range wireless communication connection, where the second working key is derived by the second electronic device based on a second periodic key by using a first derivation algorithm, the second periodic key is associated with the second user information, and the second periodic key is periodically generated by the second electronic device and sent to the first server. In this way, working keys can be sent and received between electronic devices.

In a possible implementation, the first working key is derived by the first electronic device from the first periodic key by using the first derivation algorithm, and the first working key set is derived by the first server from the first periodic key by using the first derivation algorithm. In this way, it can be ensured that an electronic device and a server use a same derivation algorithm.

In a possible implementation, after the second electronic device determines that a user of the second electronic device is a suspected target user, the method further includes: The second electronic device sends first contact information and the second user information to the first server, where the first contact information includes the first working key, the first contact information and the second user information are to be used by the first server to generate a first contact chain, and the first contact chain indicates that the user of the first electronic device was in contact with the user of the second electronic device. In this way, a contact chain can be used to assist a staff member in performing source tracing for an infection source of the target user.

In a possible implementation, before the second electronic device sends first contact information and the second user information to the first server, the method further includes: The second electronic device outputs first prompt information, where the first prompt information indicates that the user of the second electronic device is the suspected target user; and the second electronic device detects a first operation of the user. In this way, the user may be notified whether the user is a suspected target user, and if the user is a suspected target user, the user may be prompted to upload contact information.

In a possible implementation, before the second electronic device sends first contact information and the second user information to the first server, the method further includes: The second electronic device establishes a short-range wireless communication connection to a third electronic device; the second electronic device receives, through the short-range wireless communication connection, a third working key sent by the third electronic device, where the third working key is derived by the third electronic device from a third periodic key by using the first derivation algorithm, the third periodic key is periodically generated by the third electronic device and sent to the first server, the third periodic key is associated with third user information, and the third user information is sent by the third electronic device to the first server before the second electronic device receives, through the short-range wireless communication connection, the third working key sent by the third electronic device; and the second electronic device sends the second working key to the third electronic device through the short-range wireless communication connection. In this way, working keys can be sent and received between electronic devices.

According to a fourth aspect, an embodiment of this application provides an electronic device. The electronic device includes one or more processors and one or more memories. The one or more memories are coupled to the one or more processors, the one or more memories are configured to store computer program code, the computer program code includes computer instructions, and when the one or more processors execute the computer instructions, the electronic device is enabled to perform the method according to the third aspect.

According to a fifth aspect, an embodiment of this application provides a computer storage medium. The computer storage medium stores a computer program, the computer program includes program instructions, and when the program instructions are run on an electronic device, the electronic device is enabled to perform the method according to the third aspect.

### BRIEF DESCRIPTION OF DRAWINGS

FIG. 1 is a schematic diagram of an application scenario of a contact tracing method according to an embodiment of this application;
FIG. 2A to FIG. 2C are a schematic flowchart of a contact tracing method according to an embodiment of this application;
FIG. 3A to FIG. 3G are schematic diagrams of a group of user interfaces according to an embodiment of this application;
FIG. 4A to FIG. 4D are a schematic flowchart of another contact tracing method according to an embodiment of this application;
FIG. 5A to FIG. 5C are a schematic flowchart of another contact tracing method according to an embodiment of this application;
FIG. 6A and FIG. 6B are schematic diagrams of another group of user interfaces according to an embodiment of this application;
FIG. 7 is a schematic diagram of a structure of an electronic device according to an embodiment of this application;
FIG. 8 is a schematic diagram of a software structure of an electronic device according to an embodiment of this application;
FIG. 9 is a schematic diagram of a structure of another electronic device according to an embodiment of this application;
FIG. 10 is a schematic diagram of a structure of a server according to an embodiment of this application; and
FIG. 11 is a schematic diagram of a structure of another server according to an embodiment of this application.

### DESCRIPTION OF EMBODIMENTS

The following clearly and completely describes the technical solutions in embodiments of this application with reference to the accompanying drawings in embodiments of this application. In the descriptions of embodiments of this application, unless otherwise specified, "/" indicates "or". For example, A/B may indicate A or B. The term "and/or" in this specification merely describes an association relationship for describing associated objects, and indicates that three relationships may exist. For example, A and/or B may indicate the following three cases: Only A exists, both A and B exist, and only B exists. In addition, in the descriptions of embodiments of this application, "a plurality of" means two or more.

It should be understood that, the terms "first", "second", and the like in the specification, claims, and accompanying drawings of this application are used to distinguish between different objects, but are not used to describe a specific sequence. In addition, the terms "include" and "have" and any variations thereof are intended to cover non-exclusive inclusion. For example, a process, method, system, product, or device that includes a series of steps or units is not limited to the listed steps or units, but optionally further includes an unlisted step or unit, or optionally further includes another inherent step or unit for the process, method, product, or device.

The "embodiment" mentioned in this application means that specific features, structures, or characteristics described with reference to the embodiment may be included in at least one embodiment of this application. Appearance of the phrase at various locations in the specification does not necessarily refer to a same embodiment, or an independent or alternative embodiment mutually exclusive with another embodiment. A person skilled in the art explicitly and implicitly understands that the embodiments described in this application may be combined with other embodiments.

At present, in a process of preventing and controlling some infectious diseases, a person who has a history of contact with a patient of an infectious disease needs to be traced in a timely manner. In a process of tracing a contact person, a server usually needs to use some applications to obtain user information. However, a problem that uploaded user information is incomplete or inaccurate often occurs in these applications, and efficient tracing cannot be implemented.

The following describes an application scenario of a contact tracing method provided in an embodiment of this application.

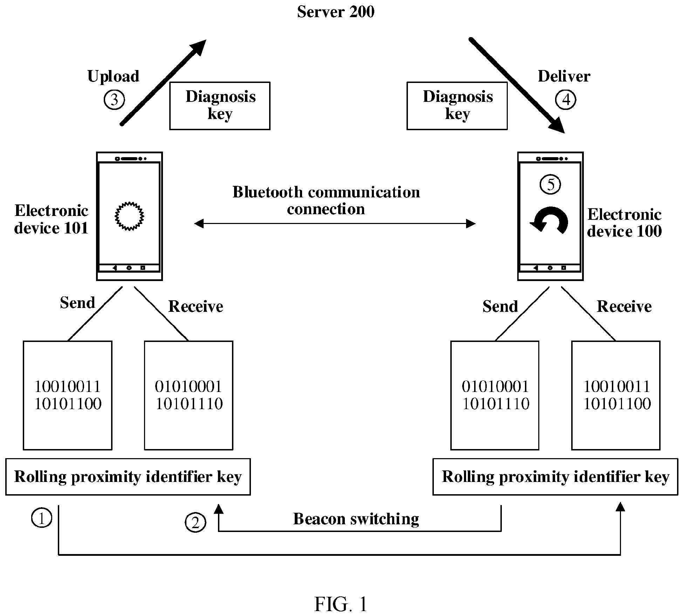

FIG. 1 shows an example of an application scenario of a contact tracing method according to an embodiment of this application.

As shown in FIG. 1, the application scenario may include an electronic device 100, an electronic device 101, and a server 200. In the contact tracing method, contact tracing for people related to an infectious disease is completed based on Bluetooth low energy. The following describes a specific implementation process of the contact tracing method.

After receiving an operation that a user downloads and installs a related contact tracing application and performs registration and login, an electronic device may generate a temporary exposure key (Temporary Exposure Key, TEK). The temporary exposure key is randomly generated, and an update cycle is 24 hours. A rolling proximity identifier key (Rolling Proximity Identifier Key, RPIK) may be derived from the temporary exposure key. The rolling proximity identifier key is used to generate a rolling proximity identifier (Rolling Proximity Identifier, RPI) shown in FIG. 1. An update cycle of the rolling proximity identifier is approximately 10 minutes, and is the same as an update cycle of a Bluetooth low energy address.

After the electronic device detects an operation of enabling Bluetooth low energy by the user, the electronic device periodically performs advertisement sending and receiving through Bluetooth low energy. For example, in a propagation range of a Bluetooth low energy signal, different electronic devices (for example, the electronic device 100 and the electronic device 101) may periodically exchange beacons through Bluetooth low energy. For example, the electronic device 101 may send a beacon to the electronic device 100, and the electronic device 100 stores the beacon after receiving the beacon. Similarly, the electronic device 100 may also send a beacon to the electronic device 101, and the electronic device 101 also stores the beacon after receiving the beacon. Information in the beacon may include information such as a rolling proximity identifier.

When a user of the electronic device 101 is determined as a target user, after receiving an operation of uploading diagnosis key (Diagnosis Keys, DK) information by the target user, the electronic device 101 may send the diagnosis key information to the server 200 (for example, a server of a public health institution). The diagnosis key information may include temporary exposure keys in a maximum of 14 days and a date on which each temporary exposure key is generated, that is, a temporary exposure key and a date of each of 14 days before a date at which the user is determined as the target user. After receiving the diagnosis key information, the server 200 may send the diagnosis key information to the electronic device 100. After receiving the diagnosis key information, the electronic device 100 may decrypt the diagnosis key information to obtain a corresponding rolling proximity identifier through calculation. If a rolling proximity identifier stored in the electronic device 100 matches the corresponding rolling proximity identifier obtained through calculation, the electronic device 100 may display prompt information, where the prompt information is used to notify that a user of the electronic device 100 is a suspected target user.

The target user may be a user suffering from an infectious disease, and the suspected target user may be a user who has a history of contact with the target user (for example, the target user and the suspected target user stayed at a place at the same time before), that is, a contact user of the target user.

It can be learned from FIG. 1 that, in the foregoing contact tracing method, a problem that uploaded user information is incomplete or inaccurate is prone to occur. In addition, the foregoing contact tracing method is completely performed on electronic devices in a distributed manner, and a contact chain cannot be generated. Consequently, the server cannot assist, based on a contact chain, a related staff member in performing source tracing for an infection source of the target user.

In this embodiment of this application, the electronic device 100 and the electronic device 101 each may also be referred to as user equipment (user equipment, UE), an access terminal, a subscriber unit, a subscriber station, a mobile station, a mobile console, a remote terminal, a mobile device, a user terminal, a terminal, a wireless communication device, a user agent, or a user apparatus. The electronic device in this embodiment of this application may be a mobile phone (mobile phone), a tablet computer (Pad), a computer with wireless sending and receiving functions, a virtual reality (virtual reality, VR) terminal device, an augmented reality (augmented reality, AR) terminal device, a handheld device, an in-vehicle device, a wearable device, or the like. This is not limited herein.

The server 200 may be a conventional server, or may be a cloud server. This is not limited herein. In this embodiment of this application, the server 200 is mainly configured to store a periodic key, deliver a working key set of a target user, generate a contact chain, and the like. The server 200 may be a server of a public health institution, and the government public health institution is responsible for establishing and maintaining the server, to complete a unified background service deployed in a centralized manner.

It should be noted that FIG. 1 describes only a schematic diagram of an application scenario of a contact tracing method by using the electronic device 100 and the electronic device 101 as an example. The schematic diagram of the application scenario may alternatively include more electronic devices. This is not limited herein.

An embodiment of this application provides a contact tracing method. Partial centralized management is used on a premise of protecting user privacy. An electronic device may automatically upload a periodic key of a user to a server for storage, and periodically derive a working key based on the periodic key. When the electronic device enables Bluetooth low energy, the electronic device may generate a Bluetooth low energy advertising packet based on the working key. Bluetooth low energy advertising packets may be sent and received between different electronic devices. When a target user appears, the server may search for a periodic key of the target user, derive all working keys based on the periodic key to obtain a working key set of the target user, and send the working key set to electronic devices of all users. The electronic device may perform contact tracing and prediction on a suspected target user based on the working key set of the target user and a previously received Bluetooth low energy advertising packet. This resolves a problem that uploaded user information is incomplete or inaccurate. In addition, after receiving contact information uploaded by the suspected target user, the server may generate a contact chain based on the contact information, to assist a related staff member in performing source tracing for an infection source of the target user.

For ease of understanding, the following describes some related concepts in embodiments of this application.

### 1. Hierarchical key management structure

A hierarchical protection method usually needs to be used for key security management. In the hierarchical key management structure, keys may be classified into two layers: a lower-layer key and an upper-layer key. The lower-layer key provides encryption protection for the upper-layer key. The hierarchical key management structure helps key management meet a corresponding specification and requirement.

### 2. Periodic key

The periodic key is located at a lower layer of a hierarchical key management structure, and is used to protect confidentiality of an upper-layer key. For example, if the upper-layer key is a working key, the periodic key may provide encryption protection for the working key, and the periodic key may be used to generate the working key by using a key derivation algorithm.

### 3. Working key

The working key is located at an upper layer of a hierarchical key management structure, is used to provide confidentiality and integrity protection for locally stored sensitive data and data that needs to be transmitted on an insecure channel, and may further provide cryptography services such as authentication and signature. The working key can be directly used by an application, and includes a key used for storage encryption, a pre-shared key, a media access control (media access control, MAC) key, a private signature key, or the like.

### 4. Trusted execution environment (Trusted Execution Environment, TEE)

The trusted execution environment is a secure environment in a mobile electronic device. A rich execution environment (Rich Execution Environment, REE) is a general-purpose execution environment in the mobile electronic device, and runs a general-purpose operating system (Operating System, OS), for example, an Android system or an iOS system. The trusted execution environment runs in an independent environment, runs in parallel with the general-purpose operating system, and provides a security service for the general-purpose operating system. The trusted execution environment has its own execution control, and has a higher security level than the general-purpose operating system. Software and hardware resources that can be accessed by the trusted execution environment are separated from those for the general-purpose operating system.

The trusted execution environment provides a secure execution environment for authorizing a trusted application (Trusted Application, TA), and also protects confidentiality, integrity, and access permission of a resource and data of the trusted application. The trusted execution environment includes an internal application programming interface and an external application programming interface. The internal application programming interface mainly includes application programming interfaces such as key management, a cryptographic algorithm, secure storage, a secure clock resource and service, and a trusted user interface. The external application programming interface is an underlying communication interface that enables a client application (Client Application, CA) running in the general-purpose operating system to access a service and data of the trusted application.

The trusted execution environment may be used in application scenarios such as content protection (for example, preventing some high-definition movies and music from being stolen), a mobile financial service (for example, mobile payment), authentication (for example, fingerprint recognition and facial recognition), and confidential information protection (for example, secure storage of a key and a certificate). For example, sensitive information such as a user identity, a key, and a certificate needs to be highly protected. The trusted execution environment may protect data and keys by using encryption and integrity protection technologies. The trusted execution environment stores sensitive information such as a user identity, a key, and a certificate in a secure area, and the sensitive information can be accessed or modified by only a trusted application authorized by the trusted execution environment. In addition, the trusted execution environment provides encryption and integrity protection mechanisms for operation processing of the sensitive information. In addition, user information, for example, sensitive information such as an address book and an SMS message, in the general-purpose execution environment may be encrypted by using the key stored in the trusted execution environment, to ensure security of the sensitive information stored in the general-purpose execution environment.

In embodiments of this application, the trusted execution environment is mainly used in an application scenario of confidential information protection.

The following describes a contact tracing method provided in an embodiment of this application.

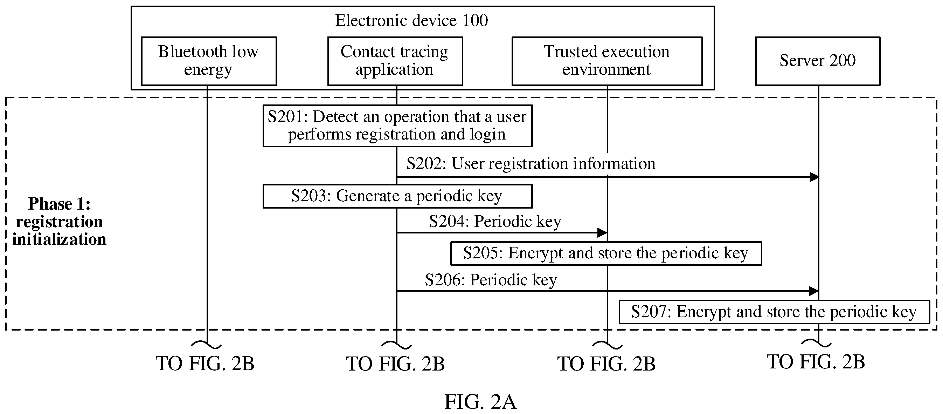

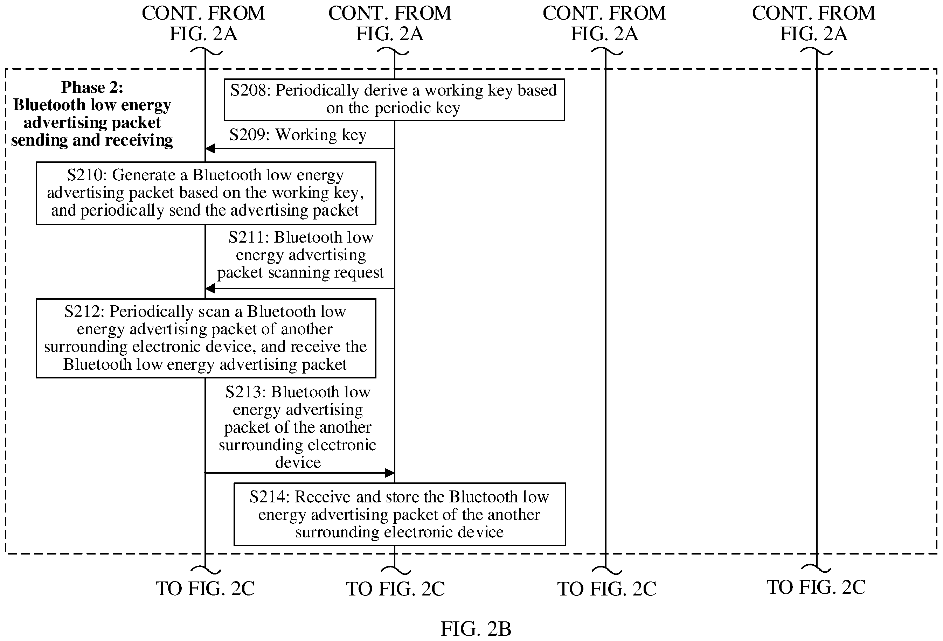

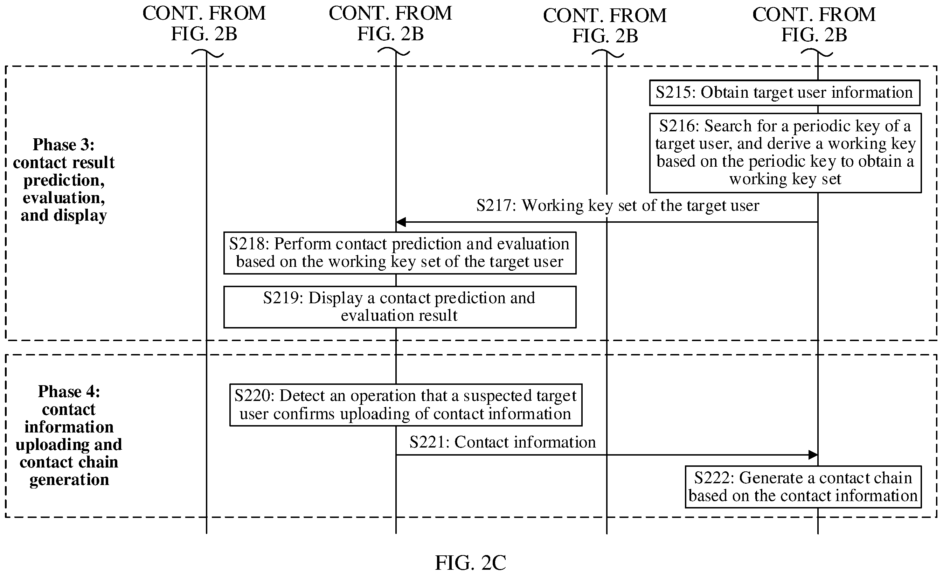

FIG. 2A to FIG. 2C show an example of a specific procedure of a contact tracing method according to an embodiment of this application.

### Application scenario: contact tracing for people related to an infectious disease

As shown in FIG. 2A to FIG. 2C, the contact tracing method may be applied to a communication system including an electronic device 100 and a server 200. The electronic device 100 may be an electronic device of a suspected target user, and the server 200 may be a server of a public service institution. The following describes in detail specific steps of the contact tracing method.

### Phase 1: registration initialization

S201 and S202: The electronic device 100 detects an operation that a user performs registration and login on a contact tracing application. In response to the operation, the electronic device 100 sends user registration information to the server 200.

Specifically, the contact tracing application may be manually downloaded and installed by the user on the electronic device 100. In a case that the contact tracing application is installed on the electronic device 100 and Bluetooth low energy has been enabled, the electronic device 100 detects the operation that the user performs registration and login on the contact tracing application, and in response to the operation, the electronic device 100 may send the user registration information to the server 200. The server 200 may store the user registration information.

The user registration information is obtained after the user completes a registration operation on the contact tracing application, and may include information such as a name, a gender, and identification information of the user.

The contact tracing application is usually an application provided by a public health institution.

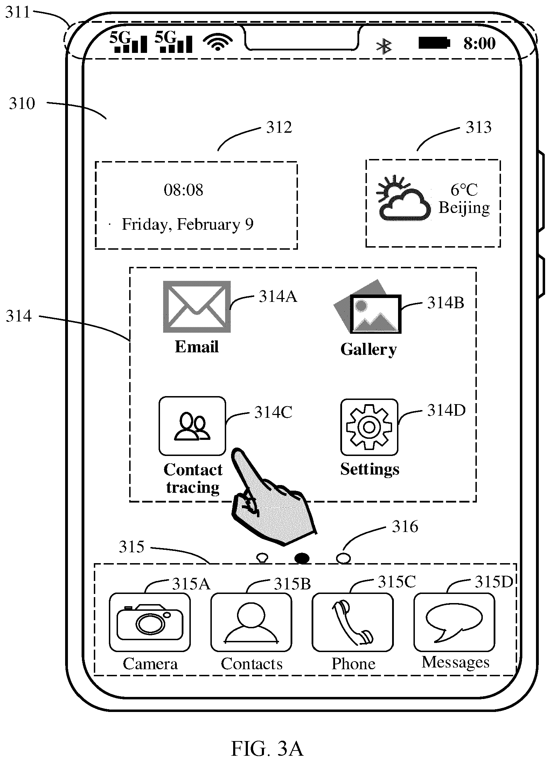

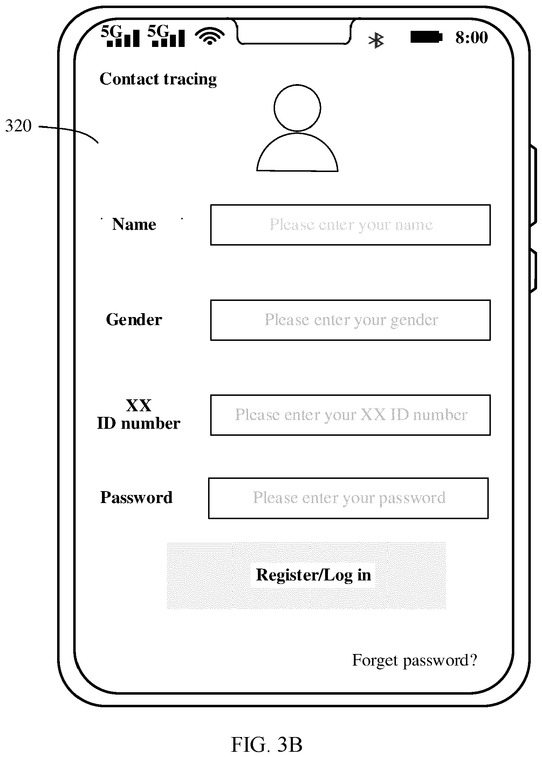

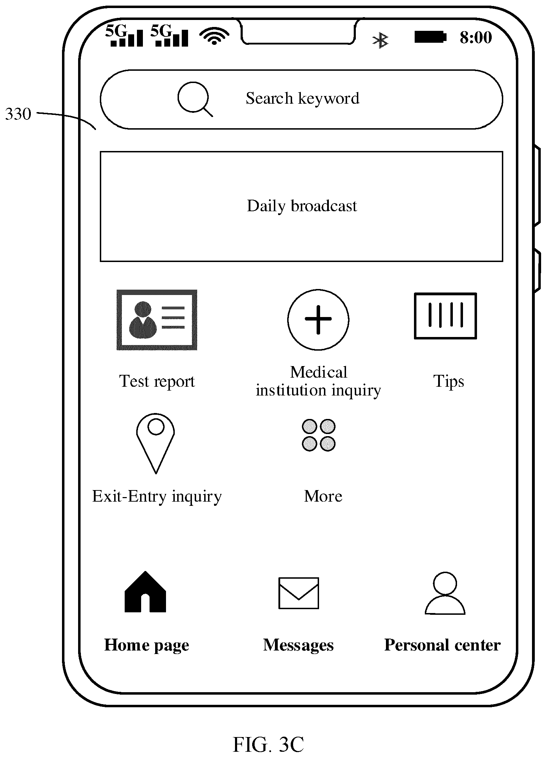

For example, FIG. 3A shows a user interface 310 in a state in which Bluetooth low energy has been enabled on the electronic device 100. The electronic device 100 may detect an operation (for example, a tap operation) performed by the user on a "contact tracing" application 314C, and in response to the operation, the electronic device 100 may display a user interface 320 shown in FIG. 3B. The electronic device 100 may detect a registration and login operation of the user (for example, an operation that the user enters "name", "gender", "XX ID number", and "password" and taps a "register/log in" option in the user interface 320). In response to the operation, the electronic device 100 may send information entered by the user to the server 200, and display a user interface 330 shown in FIG. 3C. The user interface 330 may be an interface of the contact tracing application.

In some embodiments, if the electronic device 100 does not enable Bluetooth low energy, the electronic device 100 may prompt the user to enable Bluetooth low energy.

It should be noted that the contact tracing application is an application installed on the electronic device 100. In this embodiment of this application, the contact tracing application is merely an example of a name of the application, and the application may alternatively be set to another name. This is not limited herein.

S203: The electronic device 100 generates a periodic key.

Specifically, after the electronic device 100 detects that the user completes the information registration operation, the electronic device 100 may generate the periodic key in response to the operation. The periodic key is randomly generated by the contact tracing application.

In consideration of user privacy protection, the electronic device 100 may update the periodic key. If a periodic key generated by each electronic device is fixed, because a working key in a subsequent step is generated based on the periodic key, once the periodic key is leaked, a serious privacy leakage problem may be caused.

For example, that the electronic device 100 updates the periodic key may include but is not limited to the following two implementations.

### Implementation 1

After the electronic device 100 generates a periodic key for the first time, the electronic device 100 may periodically update the periodic key. Likewise, each updated periodic key is also randomly generated. An update cycle of the periodic key may be set autonomously. For example, the update cycle may be one day, three days, five days, or the like.

### Implementation 2

After the electronic device 100 generates a periodic key for the first time, the electronic device 100 may detect an operation of opening the contact tracing application by the user, and in response to the operation, the electronic device 100 may generate a new periodic key. In other words, each time the user opens the contact tracing application, the electronic device 100 generates a new periodic key.

S204 and S205: The contact tracing application of the electronic device 100 sends the periodic key to a trusted execution environment. After receiving the periodic key, the trusted execution environment encrypts and stores the periodic key.

Specifically, after generating a periodic key for the first time and each time after updating a periodic key, the contact tracing application of the electronic device 100 sends the periodic key to the trusted execution environment. After receiving the periodic key, the trusted execution environment may encrypt and store the periodic key.

An encryption and storage principle belongs to the conventional technology. For details, refer to related data of an encryption technology. Details are not described herein.

S206 and S207: The electronic device 100 sends the periodic key to the server 200. After receiving the periodic key, the server 200 encrypts and stores the periodic key.

Specifically, after generating a periodic key for the first time and each time after updating a periodic key, the contact tracing application of the electronic device 100 sends the periodic key to the server 200. After receiving the periodic key, the server 200 may encrypt and store the periodic key. After detecting the registration operation performed by the user on the contact tracing application, the electronic device 100 may generate the periodic key (that is, the periodic key generated by the electronic device 100 for the first time), and send both the periodic key and the user registration information to the server 200. Then, when the electronic device 100 sends each updated periodic key to the server 200, a user identity may also be carried. The user identity may be generated based on the user registration information, or may be the name, the identification information, or the like in the user registration information, and indicates an electronic device of a specific registered user that sends the periodic key received by the server 200.

An encryption and storage principle belongs to the conventional technology. For details, refer to related data of an encryption technology. Details are not described herein.

In a process in which the electronic device 100 sends the periodic key to the server 200, to ensure that the periodic key is not leaked, the server 200 may generate a pair of a temporary public key and a temporary private key when the user performs registration or the periodic key is updated, and deliver the temporary public key to the electronic device 100. The temporary private key is used by the server 200 to encrypt and store the periodic key. The electronic device 100 may encrypt the periodic key by using the temporary public key, and then send an encrypted periodic key to the server 200. In this way, security of the periodic key can be ensured.

### Phase 2: Bluetooth low energy advertising packet sending and receiving

S208: The contact tracing application of the electronic device 100 derives a working key based on the periodic key.

Specifically, for example, the periodic key is updated once every three days. Within the three days, the working key may be periodically derived from the periodic key by using a key derivation algorithm, that is, new working keys are derived at intervals of a specific period of time. For example, one working key may be derived from the periodic key every day. That is, in a case that the update cycle of the periodic key is three days, three working keys may be derived from the periodic key in one update cycle.

The derivation algorithm used to derive the working key from the periodic key may be a key derivation algorithm in the conventional technology. This is not limited herein.

It should be noted that, in this embodiment of this application, that the update cycle of the periodic key is three days and an update cycle of the working key is one day is merely used as an example for description. The update cycle of the periodic key and the update cycle of the working key may alternatively be set to other values. This is not limited herein.

S209: The contact tracing application of the electronic device 100 sends the working key derived based on the periodic key to Bluetooth low energy.

Specifically, the contact tracing application of the electronic device 100 may periodically derive the working key based on the periodic key. Each time a new working key is derived, the contact tracing application of the electronic device 100 sends the new working key to the Bluetooth low energy.

S210: The Bluetooth low energy of the electronic device 100 generates a Bluetooth low energy advertising packet based on the working key, and periodically sends the advertising packet.

Specifically, after receiving the working key sent by the contact tracing application of the electronic device 100, the Bluetooth low energy of the electronic device 100 may generate the Bluetooth low energy advertising packet based on the working key.

Then, when it is detected that the Bluetooth low energy of the electronic device 100 is in an enabled state, and the contact tracing application is running in the foreground or the background, the Bluetooth low energy of the electronic device 100 may periodically send the advertising packet to another surrounding electronic device. An advertising packet sending cycle may be preset.

When Bluetooth low energy of the another surrounding electronic device is in an enabled state and is within a propagation range of a Bluetooth low energy signal, the Bluetooth low energy of the another surrounding electronic device may receive the advertising packet sent by the Bluetooth low energy of the electronic device 100, and record Bluetooth signal strength and a time point at which the advertising packet is received.

S211: The contact tracing application of the electronic device 100 sends a Bluetooth low energy advertising packet scanning request to the Bluetooth low energy.

The scanning request indicates the Bluetooth low energy of the electronic device 100 to perform the following step S212.

The scanning request may be sent only once, or may be sent periodically. This is not limited herein.

For example, if the scanning request is sent only once, the Bluetooth low energy of the electronic device 100 may always perform the following step S212 without stopping. For another example, if the scanning request is periodically sent in a cycle of 5 minutes, the Bluetooth low energy of the electronic device 100 may perform the following step S212 for a period of time (for example, 3 minutes), then stop, and continue to perform the following step S212 after the scanning request is received next time.

S212: The Bluetooth low energy of the electronic device 100 periodically scans a Bluetooth low energy advertising packet sent by the another surrounding electronic device, and receives the Bluetooth low energy advertising packet.

Specifically, after receiving the Bluetooth low energy advertising packet scanning request sent by the contact tracing application of the electronic device 100, the Bluetooth low energy of the electronic device 100 may periodically scan the Bluetooth low energy advertising packet sent by the another surrounding electronic device. The Bluetooth low energy advertising packet is generated based on a working key of the another surrounding electronic device.

Then, the Bluetooth low energy of the electronic device 100 may receive a found Bluetooth low energy advertising packet sent by the another surrounding electronic device, and record Bluetooth signal strength and a time point at which the advertising packet is received. An advertising packet scanning cycle may be preset.

S213: The Bluetooth low energy of the electronic device 100 sends the Bluetooth low energy advertising packet of the another surrounding electronic device to the contact tracing application of the electronic device 100.

Specifically, after receiving the Bluetooth low energy advertising packet sent by the another surrounding electronic device, the Bluetooth low energy of the electronic device 100 may send the Bluetooth low energy advertising packet of the another surrounding electronic device to the contact tracing application of the electronic device 100. In addition, the Bluetooth low energy of the electronic device 100 may also send, to the contact tracing application of the electronic device 100, the Bluetooth signal strength and the time point at which the advertising packet is received.

S214: The contact tracing application of the electronic device 100 receives and stores the Bluetooth low energy advertising packet of the another surrounding electronic device.

Specifically, after receiving the Bluetooth low energy advertising packet of the another surrounding electronic device that is sent by the Bluetooth low energy of the electronic device 100, the contact tracing application of the electronic device 100 may store the Bluetooth low energy advertising packet. In addition, the contact tracing program of the electronic device 100 may also store the Bluetooth signal strength and the time point at which the advertising packet is received that are sent by the Bluetooth low energy of the electronic device 100.

### Phase 3: contact result prediction, evaluation, and display

S215: The server 200 obtains target user information.

Specifically, the server 200 may obtain the target user information from a public service institution in real time. The server 200 may be a server of the public service institution, and the target user information may include information such as a name, a gender, and identification information of a target user.

In a possible implementation, the server 200 may obtain all target user information once from the public service institution in a centralized manner at intervals of a period of time. For example, the server 200 may obtain all target user information in one previous hour once from the public service institution every hour. For another example, the server 200 may obtain all target user information in one previous day once from the public service institution every day.

S216 and S217: The server 200 searches for a periodic key of the target user, derives a working key based on the periodic key to obtain a working key set, and then sends the working key set of the target user to the electronic device 100.

Specifically, the server 200 may find the periodic key of the target user based on the target user information, and derive all working keys based on the periodic key. All the working keys may form one working key set. The periodic key may be all periodic keys generated by the target user in last N days, where N is a positive integer, and a value of N may be set autonomously. For example, N may be set to 14. For another example, N may be set to 21. A key derivation algorithm used by the server 200 to derive the working key based on the periodic key is the same as the key derivation algorithm used by the electronic device to derive the working key based on the periodic key. Then, the server 200 may send the working key set of the target user to electronic devices of all users, or may send the working key set of the target user to all electronic devices (for example, the electronic device 100) that register with and log in to the contact tracing application and that are other than an electronic device of the target user. The working key set of the target user may include all working keys derived by the server 200 based on the periodic key of the target user.

For example, the electronic device of the target user may be an electronic device 101. The contact tracing application is installed and Bluetooth low energy is enabled on the electronic device 101. Before the target user is determined as the target user, the electronic device 101 detects an operation that the user performs registration and login on the contact tracing application. In response to the operation, the electronic device 101 may send user registration information to the server 200, and the server 200 may store the user registration information. The user registration information may include information such as a name, a gender, and identification information of the target user. The electronic device 101 may also send a generated periodic key to the server 200. After the target user is determined as the target user, the server 200 may find the user registration information of the target user on the contact tracing application based on the obtained target user information. Then, the server 200 may find the periodic key of the target user based on the user registration information. For example, the server 200 may find all periodic keys generated in 14 days before the target user is determined as the target user, that is, each periodically generated periodic key in 14 days before a date on which the target user is determined as the target user. Then, the server 200 may derive the working key based on the periodic key. That is, a corresponding working key may be derived from each periodic key by using a key derivation algorithm. Then, the server 200 may send the working key set of the target user to the electronic device 100. The working key set of the target user includes all working keys derived based on the periodic key of the target user.

In some embodiments of this application, the working key set that is of the target user and that is sent by the server 200 to the electronic device 100 may alternatively be working key sets of a plurality of target users. In other words, the server 200 may simultaneously send the working key sets of the plurality of target users to the electronic device 100.

In some embodiments of this application, the foregoing Bluetooth advertising packet may alternatively be generated by the electronic device based on a key derived from the working key. For example, the electronic device may periodically derive the working key based on the periodic key by using the key derivation algorithm. It is assumed that an update cycle of the working key is one day. Within one day, the electronic device may periodically derive a plurality of keys based on the working key by using a same key derivation algorithm, that is, may derive a new key at intervals of a period of time (for example, at intervals of 10 minutes). The electronic device may generate a Bluetooth advertising packet based on the key derived from the working key, and advertise the Bluetooth advertising packet, and may also receive and store a Bluetooth advertising packet advertised by another surrounding electronic device. Compared with generating a Bluetooth advertising packet based on the working key, generating the Bluetooth advertising packet based on the key derived from the working key can improve security and enhance user privacy protection. Similarly, after finding the periodic key of the target user, the server 200 may first derive all working keys based on the periodic key of the target user by using the same key derivation algorithm, and send all the working keys to electronic devices of all users. Then, the electronic devices of all the users perform derivation based on all the working keys by using the same key derivation algorithm. Then, subsequent contact prediction and evaluation are completed based on derived keys and a previously stored key for generating a Bluetooth low energy advertising packet.

S218 and S219: The electronic device 100 performs contact prediction and evaluation based on the working key set of the target user, and after the prediction and evaluation are completed, the electronic device 100 displays a contact prediction and evaluation result.

Specifically, after receiving the working key set of the target user sent by the server 200, the electronic device 100 may perform contact prediction and evaluation based on the working key set of the target user, to determine whether the user of the electronic device 100 was in contact with the target user, and calculate information such as contact time and a contact distance.

A specific implementation of contact prediction and evaluation is as follows: The electronic device 100 parses and calculates all previously stored Bluetooth low energy advertising packets of another surrounding electronic device to determine whether a working key for generating the Bluetooth low energy advertising packets matches the working key set of the target user sent by the server 200. If matching fails (that is, the working key for generating the Bluetooth low energy advertising packets matches none of all working keys in the working key set of the target user sent by the server 200), it indicates that the user of the electronic device 100 has not been in contact with the target user, and the user of the electronic device 100 is not a suspected target user. If matching succeeds (that is, the working key for generating the Bluetooth low energy advertising packets matches at least one working key in the working key set of the target user sent by the server 200), it indicates that the user of the electronic device 100 was in contact with the target user, and the user of the electronic device 100 is a suspected target user. Further, the electronic device 100 may identify contact time between the user of the electronic device 100 and the target user based on a recorded time point at which the advertising packet is received, and calculate a contact distance between the user of the electronic device 100 and the target user based on recorded Bluetooth signal strength. The contact distance may be a distance range calculated by the electronic device 100 based on the Bluetooth signal strength and the contact time. Areal contact distance may be included in the distance range, and the contact distance may not be a specific value.

After the electronic device 100 completes the contact prediction and evaluation, the electronic device 100 may display the contact prediction and evaluation result. The contact prediction and evaluation result may include information such as information indicating whether the user of the electronic device 100 was in contact with the target user, contact time, and a contact distance. If the user of the electronic device 100 was in contact with the target user, that is, the user of the electronic device 100 is a suspected target user, the contact prediction and evaluation result may further include prompt information about whether the user confirms uploading of contact information. The prompt information is used to prompt the suspected target user to upload the contact information.

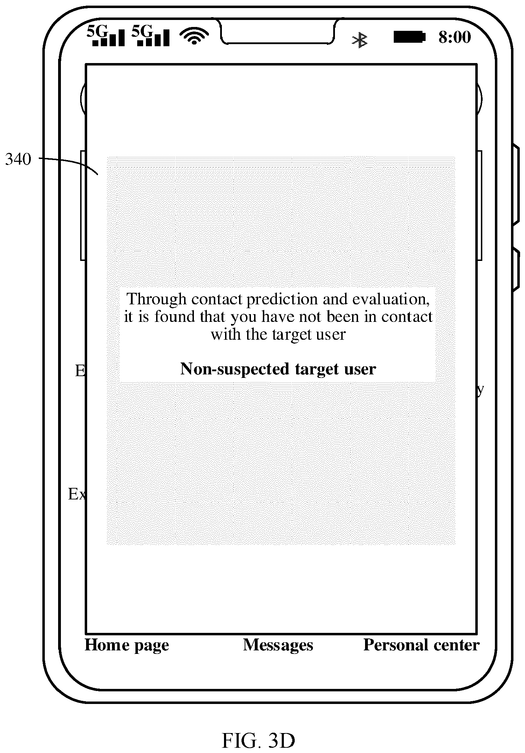

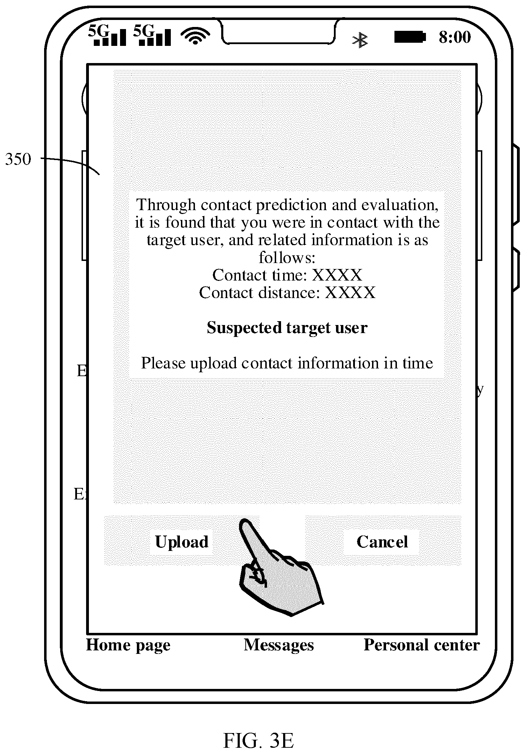

For example, if the user of the electronic device 100 is not a suspected target user, the electronic device 100 may display a pop-up window 340 shown in FIG. 3D. The pop-up window 340 displays a contact prediction and evaluation result, and the contact prediction and evaluation result indicates that the user of the electronic device 100 is not a suspected target user. If the user of the electronic device 100 is a suspected target user, the electronic device 100 may display a pop-up window 350 shown in FIG. 3E. The pop-up window 350 displays a contact prediction and evaluation result, and the contact prediction and evaluation result indicates that the user of the electronic device 100 is a suspected target user, and is used to prompt the suspected target user to upload contact information.

Optionally, if the user of the electronic device 100 is not a suspected target user, the electronic device 100 may alternatively not display the pop-up window 340, that is, the electronic device 100 does not need to display any related information after the contact prediction and evaluation.

It should be noted that all steps in the phase 1, the phase 2, and the phase 3 are described by using only an example in which the electronic device 100 is an electronic device of a suspected target user. Not limited to the electronic device of the suspected target user, electronic devices of all users (for example, the target user and a non-suspected target user) may perform all the steps in the phase 1, the phase 2, and the phase 3. This is not limited in this embodiment of this application.

### Phase 4: contact information uploading and contact chain generation

S220 and S221: The electronic device 100 detects an operation that the suspected target user confirms uploading of the contact information. In response to the operation, the electronic device 100 sends the contact information to the server 200.

Specifically, if the user of the electronic device 100 is a suspected target user, after the electronic device 100 displays the contact prediction and evaluation result, the electronic device 100 may detect an operation that the suspected target user confirms uploading of contact information (for example, a tap operation performed by the user on an "upload" option shown in FIG. 3E). In response to the operation, the electronic device 100 may send the contact information to the server 200. The contact information may include information such as the working key of the target user that was in contact with the electronic device 100, contact time, and a contact distance.

S222: The server 200 generates a contact chain based on the contact information.

Specifically, a key association storage model is built in the server 200. After the server 200 receives contact information sent by an electronic device (for example, the electronic device 100) of a suspected target user, the server may determine an association relationship between working keys of users based on the contact information, and store the association relationship. Then, the server 200 may generate a contact chain through deduction based on the stored association relationship, to assist a staff member in performing source tracing for an infection source of the target user.

It is easy to understand that there may be one or more suspected target users, and the contact information may be contact information sent by electronic devices of all suspected target users to the server 200.

Contact chain generation is described below by using an example.

It is assumed that a user 1 was in contact with a user 2 and a user 3, and the user 2 was in contact with a user 4. After the user 1 is determined as a target user, both the user 2 and the user 3 become suspected target users. After the user 2 is also determined as a target user through contact isolation observation, the user 4 also becomes a suspected target user (or after an electronic device of the user 2 sends contact information to the server 200, the user 4 becomes a contact user of a suspected target user). Therefore, a contact chain of user 1->user 2->user 4 is formed. A specific implementation of generating the contact chain is as follows: After the user 1 is determined as the target user, the server 200 may determine, based on working keys in contact information uploaded by all suspected target users, that there is an association relationship between a working key of the user 1 and both a working key of the user 2 and a working key of the user 3, and store the association relationship as an association relationship 1. After the user 2 is also determined as the target user through contact isolation observation, the server 200 may determine, based on working keys in contact information uploaded by all suspected target users who were in contact with the user 2, that there is an association relationship between the working key of the user 2 and a working key of the user 4, and store the association relationship as an association relationship 2. The server 200 may form the contact chain of user 1->user 2->user 4 based on the association relationship 1 and the association relationship 2. By analogy, one contact chain may include n users, where n is a positive integer greater than 1.

After the contact chain is generated, the server 200 may perform source tracing for the infection source of the target user based on the contact chain. For example, the server 200 may find, based on the contact chain, a user who was first in contact with the target user, and then may find the infection source of the target user with reference to an analysis result obtained after a staff member of the public service institution performs comprehensive analysis based on detection information such as an environment sample and a physiological data sample (for example, a respiratory sample or a blood sample) of the user who was first in contact with the target user, to complete source tracing.

In a possible implementation, considering that some suspected target users may be unwilling to upload contact information, or some suspected target users fail to upload contact information in a timely manner, contact information received by the server 200 within a period of time after the server 200 completes sending the working key set of the target user (that is, completes performing step S217) may be considered as contact information sent by electronic devices carried by all suspected target users to the server 200, or may be considered as valid contact information. Contact information received by the server 200 after the foregoing period of time may be considered as invalid contact information, and the server 200 does not generate a contact chain based on the invalid contact information. The period of time may be 1 hour, 2 hours, or the like. This is not limited herein.

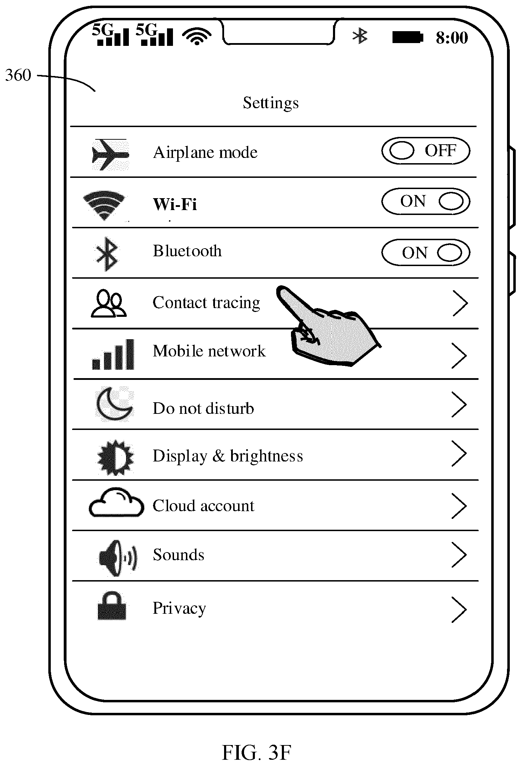

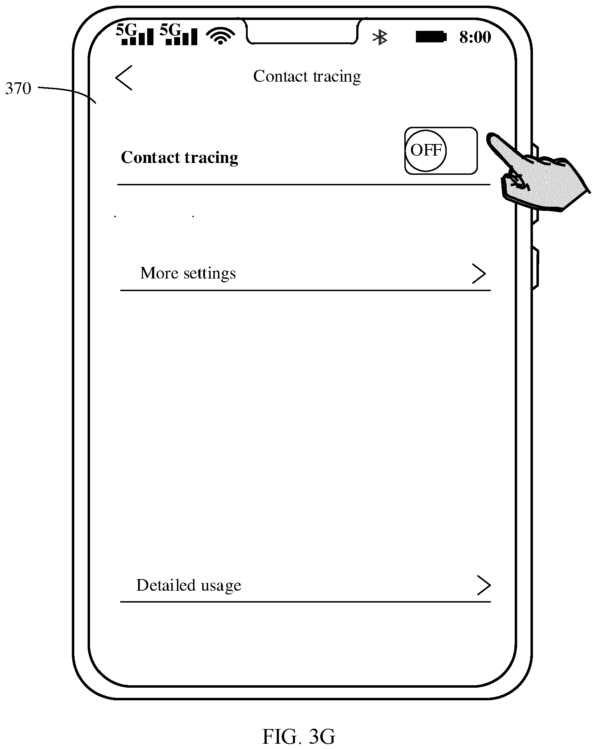

It should be noted that, in this embodiment of this application, the foregoing contact tracing method is implemented by using the foregoing contact tracing application as an example. This is not limited. The foregoing contact tracing method may alternatively be implemented by using an applet in some applications (that is, another application invoked by using the some applications), a background service, or the like. This is limited in this embodiment of this application. For example, the foregoing contact tracing function may alternatively be implemented in a "settings" application. As shown in FIG. 3F, the electronic device 100 may detect a tap operation of the user on a "contact tracing" option, and in response to the operation, the electronic device 100 may display a user interface 370 shown in FIG. 3G. Then, the electronic device 100 may detect a tap operation of turning on a "contact tracing" switch control by the user, and in response to the operation, the electronic device 100 may enable the contact tracing function.

This embodiment of this application provides the contact tracing method. Partial centralized management is used on a premise of protecting user privacy. An electronic device may automatically upload a periodic key of a user to a server for storage, and periodically derive a working key based on the periodic key. When the electronic device enables Bluetooth low energy, the electronic device may generate a Bluetooth low energy advertising packet based on the working key. Bluetooth low energy advertising packets may be sent and received between different electronic devices. When a target user appears, the server may search for a periodic key of the target user, derive all working keys based on the periodic key to obtain a working key set of the target user, and send the working key set to electronic devices of all users. The electronic device may perform contact tracing and prediction on a suspected target user based on the working key set of the target user and a previously received Bluetooth low energy advertising packet. This resolves a problem that uploaded user information is incomplete or inaccurate. In addition, after receiving contact information uploaded by the suspected target user, the server may generate a contact chain based on the contact information, to assist a related staff member in performing source tracing for an infection source of the target user.

It should be noted that, in this embodiment of this application, only the application scenario of contact tracing for people related to an infectious disease is used as an example to describe the specific procedure of the contact tracing method in detail. Not limited to the application scenario of contact tracing for people related to an infectious disease, the contact tracing method provided in this embodiment of this application is also applicable to another application scenario (for example, lost property search). This is not limited herein.

The following describes another contact tracing method provided in an embodiment of this application.

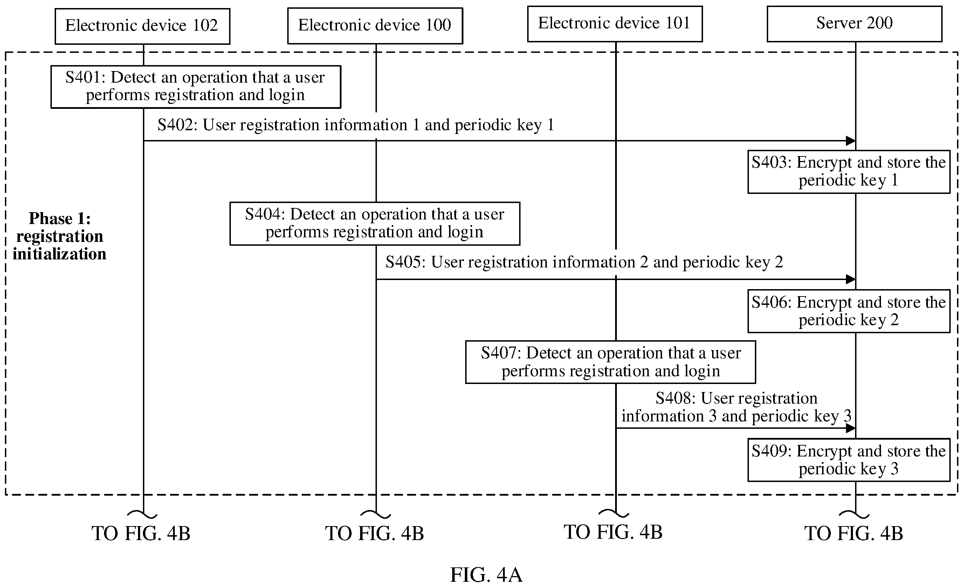

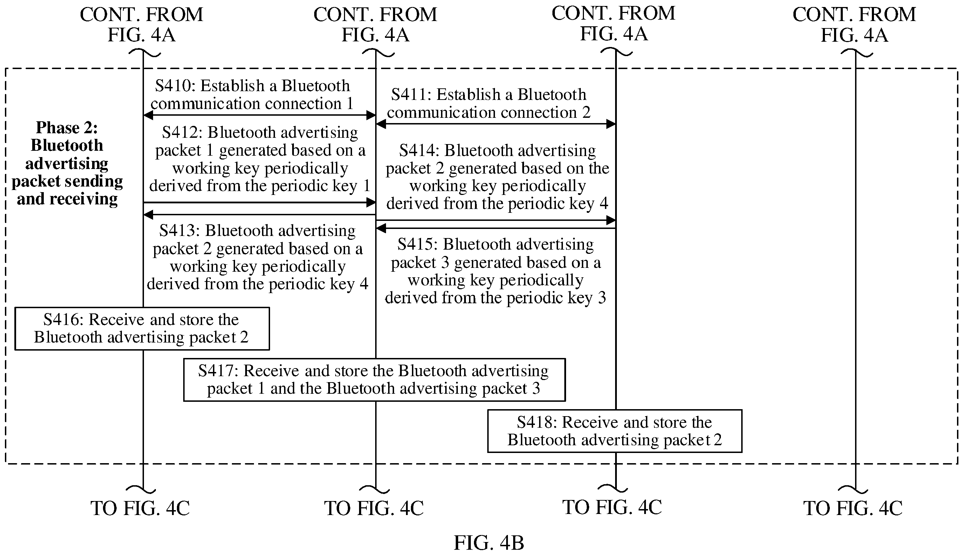

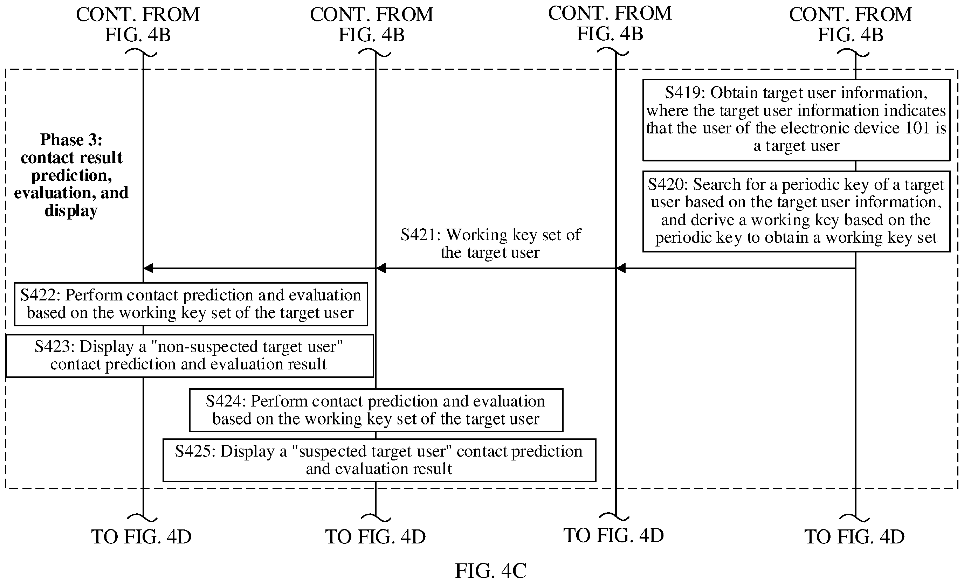

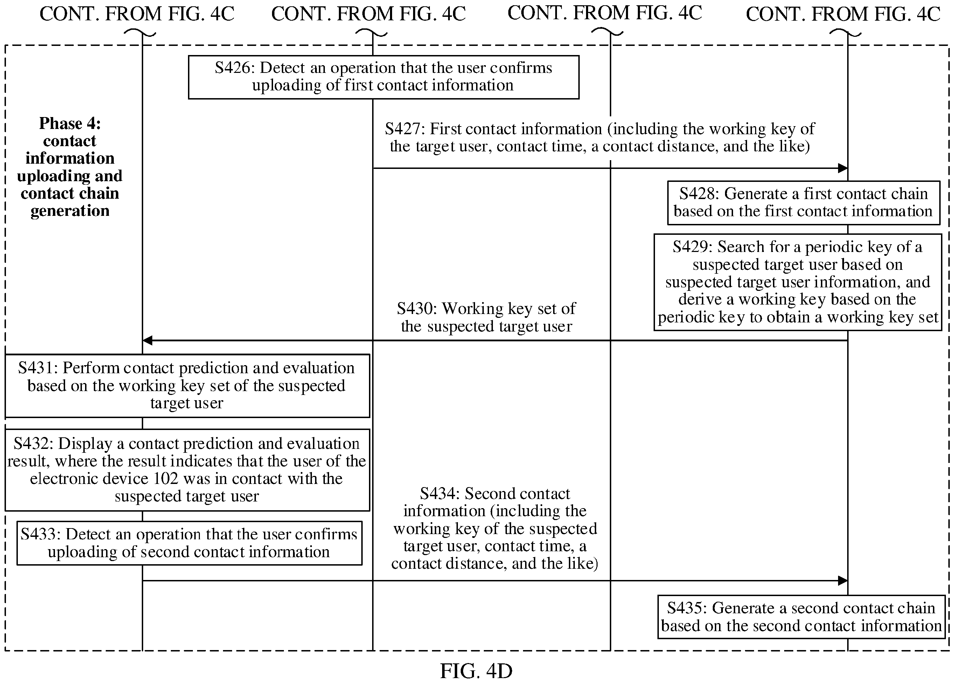

FIG. 4A to FIG. 4D show an example of a specific procedure of another contact tracing method according to an embodiment of this application.

### Application scenario: contact tracing for people related to an infectious disease

As shown in FIG. 4A to FIG. 4D, the contact tracing method may be applied to a communication system including an electronic device 100, an electronic device 101, an electronic device 102, and a server 200. The server 200 may be a server of a public service institution. On each of the electronic device 100, the electronic device 101, and the electronic device 102, a contact tracing application is installed, and Bluetooth low energy is already in an enabled state. The following describes in detail specific steps of the contact tracing method.

### Phase 1: registration initialization

S401 to S403: The electronic device 102 detects an operation that a user performs registration and login. In response to the operation, the electronic device 102 sends user registration information 1 and a periodic key 1 to the server 200. After receiving the user registration information 1 and the periodic key 1, the server 200 may encrypt and store the periodic key 1 and store the user registration information 1.

S404 to S406: The electronic device 100 detects an operation that a user performs registration and login. In response to the operation, the electronic device 100 sends user registration information 2 and a periodic key 2 to the server 200. After receiving the user registration information 2 and the periodic key 2, the server 200 may encrypt and store the periodic key 2 and store the user registration information 2.

S407 to S409: The electronic device 101 detects an operation that a user performs registration and login. In response to the operation, the electronic device 101 sends user registration information 3 and a periodic key 3 to the server 200. After receiving the user registration information 3 and the periodic key 3, the server 200 may encrypt and store the periodic key 3 and store the user registration information 3.

The user registration information 1, the user registration information 2, and the user registration information 3 each may include information such as a name, a gender, and identification information of the user. The periodic key 1, the periodic key 2, and the periodic key 3 each may be periodically updated and sent to the server 200.

In this embodiment of this application, for a specific process of performing step S404 to step S406 by the electronic device 100, refer to related content in the phase 1 in the embodiment in FIG. 2A to FIG. 2C. A specific process of performing step S407 to step S409 by the electronic device 101 and a specific process of performing step S401 to step S403 by the electronic device 102 are similar to the specific process of performing step S404 to step S406 by the electronic device 100. Details are not described herein again.

A time sequence in which the electronic device 100, the electronic device 101, and the electronic device 102 complete the registration initialization in the phase 1 is not limited in this embodiment of this application.

### Phase 2: Bluetooth advertising packet sending and receiving

S410: The electronic device 100 establishes a Bluetooth communication connection 1 to the electronic device 102.

S411: The electronic device 100 establishes a Bluetooth communication connection 2 to the electronic device 101.

S412: The electronic device 102 sends, to the electronic device 100 through the Bluetooth communication connection 1, a Bluetooth advertising packet 1 generated based on a working key periodically derived from the periodic key 1.

S413: The electronic device 100 sends, to the electronic device 102 through the Bluetooth communication connection 1, a Bluetooth advertising packet 2 generated based on a working key periodically derived from the periodic key 2.

S414: The electronic device 100 sends, to the electronic device 101 through the Bluetooth communication connection 2, the Bluetooth advertising packet 2 generated based on the working key periodically derived from the periodic key 2.

S415: The electronic device 101 sends, to the electronic device 100 through the Bluetooth communication connection 2, a Bluetooth advertising packet 3 generated based on a working key periodically derived from the periodic key 3.

S416: The electronic device 102 receives and stores the Bluetooth advertising packet 2.

S417: The electronic device 100 receives and stores the Bluetooth advertising packet 1 and the Bluetooth advertising packet 3.

S418: The electronic device 101 receives and stores the Bluetooth advertising packet 2.

The Bluetooth advertising packet 1, the Bluetooth advertising packet 2, and the Bluetooth advertising packet 3 each may be periodically sent, received, and stored.

In this embodiment of this application, for a specific process of sending, receiving, and storing Bluetooth advertising packets between the electronic device 102 and the electronic device 100, and a specific process of sending, receiving, and storing Bluetooth advertising packets between the electronic device 101 and the electronic device 100, refer to related content in the phase 2 in the embodiment in FIG. 2A to FIG. 2C. Details are not described herein again.

In this embodiment of this application, a time sequence of Bluetooth advertising packet sending and receiving between the electronic device 102 and the electronic device 100 and Bluetooth advertising packet sending and receiving between the electronic device 101 and the electronic device 100 is not limited.

### Phase 3: contact result prediction, evaluation, and display

S419: The server 200 obtains target user information, where the target user information indicates that the user of the electronic device 101 is a target user.

S420: The server 200 searches for a periodic key of the target user based on the target user information, and derives a working key based on the periodic key to obtain a working key set.

S421: The server 200 sends the working key set of the target user to the electronic device 100, the electronic device 101, and the electronic device 102 at the same time.

S422 and S423: The electronic device 102 performs contact prediction and evaluation based on the working key set of the target user, and displays a "non-suspected target user" contact prediction and evaluation result.

The contact prediction and evaluation result for the user of the electronic device 102 may display "non-suspected target user" related information, which indicates that the user of the electronic device 102 is a non-suspected target user, that is, the user of the electronic device 102 has not been in contact with the target user.

S424 and S425: The electronic device 100 performs contact prediction and evaluation based on the working key set of the target user, and displays a "suspected target user" contact prediction and evaluation result. The contact prediction and evaluation result indicates that the user was in contact with the target user.

The contact prediction and evaluation result for the user of the electronic device 100 may display "suspected target user" related information, which indicates that the user of the electronic device 100 is a suspected target user, that is, the user of the electronic device 100 was in contact with the target user.

Specifically, that the electronic device 100 performs contact prediction and evaluation based on the working key set of the target user may include: The electronic device 100 parses and calculates a received and stored Bluetooth advertising packet (including the Bluetooth advertising packet 1 and the Bluetooth advertising packet 3), and if the electronic device 100 determines that the working key set of the target user includes one or more working keys for generating the Bluetooth advertising packet, the user of the electronic device 100 is a suspected target user. Because the user of the electronic device 101 is the target user, and the working key set of the user of the electronic device 101 includes the working key for generating the Bluetooth advertising packet 3, the user of the electronic device 100 is a suspected target user.

In this embodiment of this application, a time sequence in which the electronic device 102 and the electronic device 100 complete the contact result prediction, evaluation, and display in the phase 3 is not limited.

### Phase 4: contact information uploading and contact chain generation

S426 and S427: After detecting an operation that the user confirms uploading of first contact information, the electronic device 100 sends the first contact information to the server 200, where the first contact information may include information such as the working key of the target user, contact time, and a contact distance.

S428: The server 200 generates a first contact chain based on the first contact information.

After the first contact information is received, because the server 200 stores the working key set of the target user, the server 200 may determine that the working key of the target user in the first contact information is the working key of the user of the electronic device 101. In addition, because a user identity is carried when the electronic device 100 sends the contact information to the server 200, the server 200 may determine, based on the user identity, that the first contact information is sent by the electronic device 100. Therefore, the server 200 may determine that there is an association relationship between the user of the electronic device 101 and the user of the electronic device 100, that is, there is an association relationship between the working key of the user of the electronic device 101 and the working key of the user of the electronic device 100, and store the association relationship as an association relationship 1. The server 200 may generate, based on the association relationship 1, the first contact chain of the user of the electronic device 101->the user of the electronic device 100. The first contact chain indicates that the user of the electronic device 101 was in contact with the user of the electronic device 100.

S429: The server 200 searches for a periodic key of a suspected target user based on suspected target user information, and derives a working key based on the periodic key to obtain a working key set.

S430: The server sends the working key set of the suspected target user to the electronic device 102.

S431 and S432: The electronic device 102 performs contact prediction and evaluation based on the working key set of the suspected target user, and displays a contact prediction and evaluation result, where the result indicates that the user of the electronic device 102 was in contact with the suspected target user.

S433 and S434: After detecting an operation that the user confirms uploading of second contact information, the electronic device 102 sends the second contact information to the server 200, where the second contact information may include information such as the working key of the suspected target user, contact time, and a contact distance.

S435: The server 200 generates a second contact chain based on the second contact information.

After the second contact information is received, because the server 200 stores the working key set of the suspected target user, the server 200 may determine that the working key of the suspected target user in the second contact information is the working key of the user of the electronic device 100. In addition, because a user identity is carried when the electronic device 102 sends the second contact information to the server 200, the server 200 may determine, based on the user identity, that the second contact information is sent by the electronic device 102. Therefore, the server 200 may determine that there is an association relationship between the user of the electronic device 102 and the user of the electronic device 100, that is, there is an association relationship between the working key of the user of the electronic device 102 and the working key of the user of the electronic device 100, and store the association relationship as an association relationship 2. The server 200 may generate, based on the first contact chain and the association relationship 2, the second contact chain of the user of the electronic device 101 ->the user of the electronic device 100->the user of the electronic device 102. The second contact chain indicates that the user of the electronic device 102 was in contact with the user of the electronic device 100.

The following describes another contact tracing method provided in an embodiment of this application.

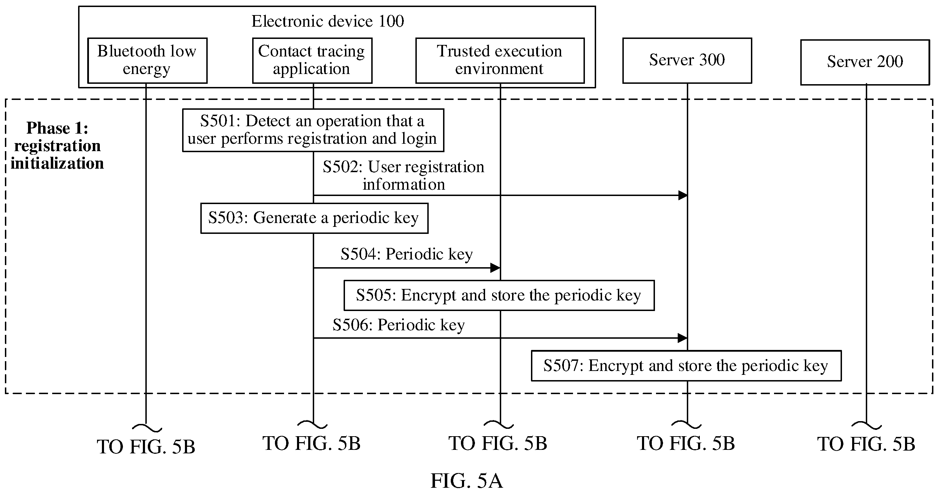

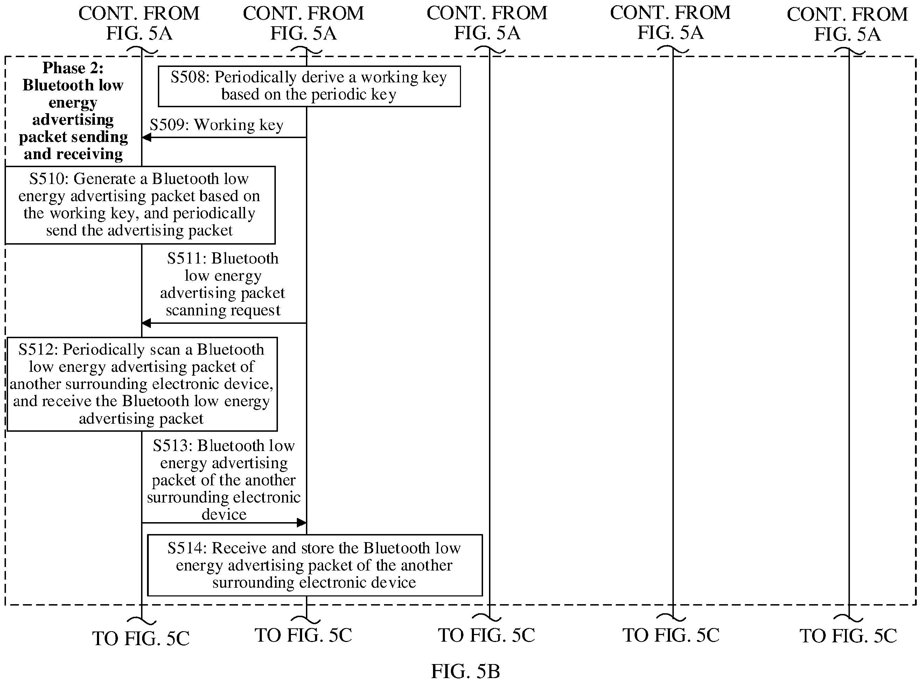

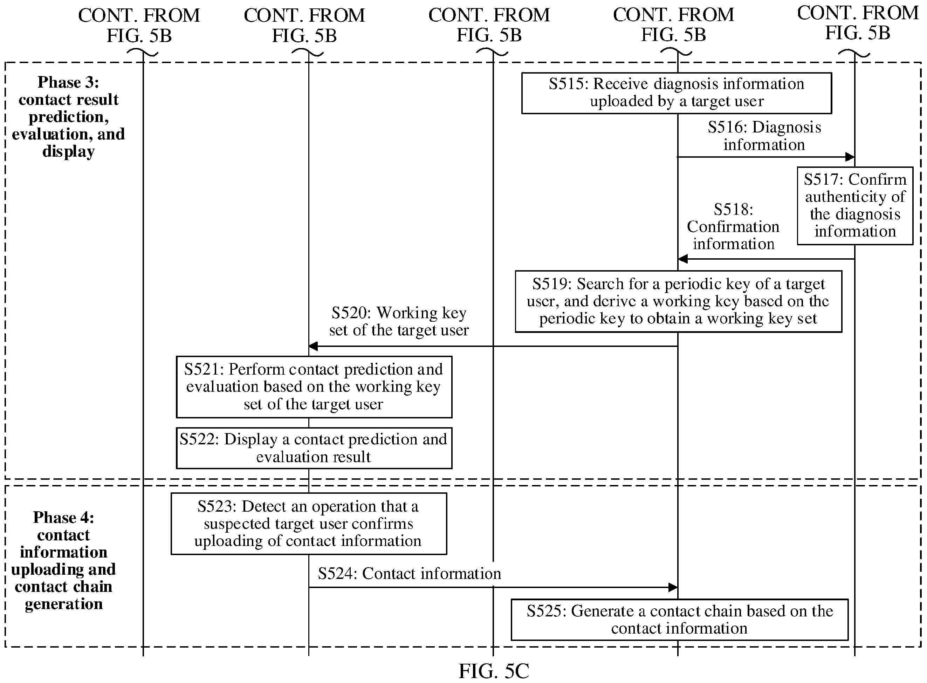

FIG. 5Ato FIG. 5C show an example of a specific procedure of another contact tracing method according to an embodiment of this application.

### Application scenario: contact tracing for people related to an infectious disease

As shown in FIG. 5A to FIG. 5C, the contact tracing method may be applied to a communication system including an electronic device 100, a server 200, and a server 300. The electronic device 100 may be an electronic device of a suspected target user, the server 200 may be a server of a public service institution, and the server 300 may be a server of a contact tracing application. The following describes in detail specific steps of the contact tracing method.

### Phase 1: registration initialization

S501 and S502: The electronic device 100 detects an operation that a user performs registration and login on a contact tracing application. In response to the operation, the electronic device 100 sends user registration information to the server 300.

S503: The electronic device 100 generates a periodic key.

S504 and S505: The contact tracing application of the electronic device 100 sends the periodic key to a trusted execution environment. After receiving the periodic key, the trusted execution environment encrypts and stores the periodic key.

S506 and S507: The electronic device 100 sends the periodic key to the server 300. After receiving the periodic key, the server 300 encrypts and stores the periodic key.

For a specific execution process of step S501 to step S507, refer to related content in step S201 to step S207 in the embodiment shown in FIG. 2A to FIG. 2C. Details are not described herein again.

### Phase 2: Bluetooth low energy advertising packet sending and receiving

S508: The contact tracing application of the electronic device 100 periodically derives a working key based on the periodic key.

S509: The contact tracing application of the electronic device 100 sends the working key periodically derived based on the periodic key to Bluetooth low energy.

S510: The Bluetooth low energy of the electronic device 100 generates a Bluetooth low energy advertising packet based on the working key, and periodically sends the advertising packet.

S511: The contact tracing application of the electronic device 100 sends a Bluetooth low energy advertising packet scanning request to the Bluetooth low energy.

S512: The Bluetooth low energy of the electronic device 100 periodically scans a Bluetooth low energy advertising packet sent by another surrounding electronic device, and receives the Bluetooth low energy advertising packet.

S513: The Bluetooth low energy of the electronic device 100 sends the Bluetooth low energy advertising packet of the another surrounding electronic device to the contact tracing application of the electronic device 100.

S514: The contact tracing application of the electronic device 100 receives and stores the Bluetooth low energy advertising packet of the another surrounding electronic device.

For a specific execution process of step S508 to step S514, refer to related content in step S208 to step S214 in the embodiment shown in FIG. 2A to FIG. 2C. Details are not described herein again.

### Phase 3: contact result prediction, evaluation, and display

S515 and S516: The server 300 receives target user information uploaded by a target user, and then sends the target user information to the server 200.

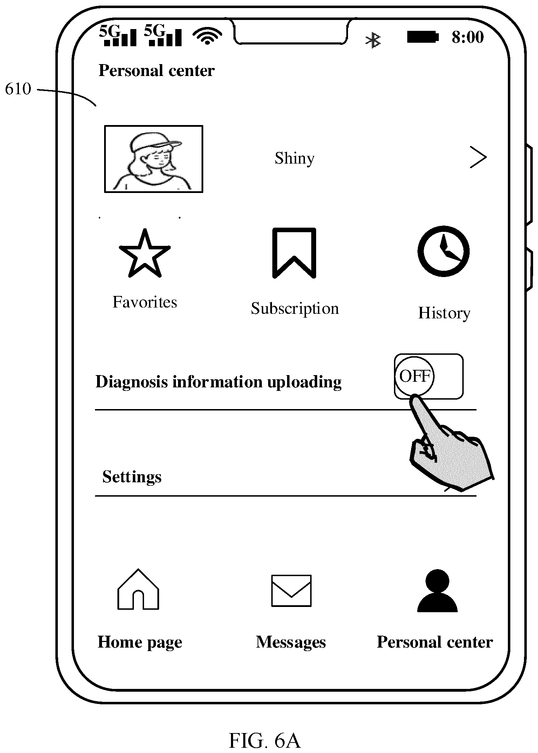

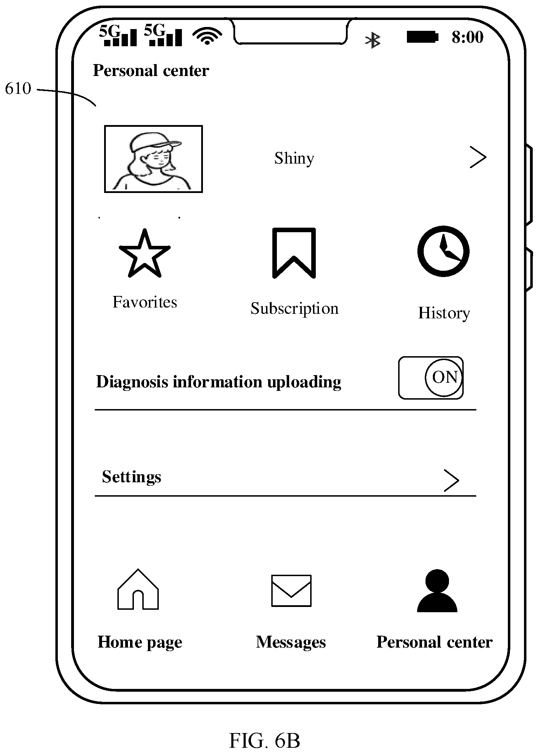

Specifically, an electronic device of the target user may be an electronic device 101. The contact tracing application is installed and Bluetooth low energy is enabled on the electronic device 101. If the target user has registered with and logged in to the contact tracing application before being determined as the target user, the electronic device 101 may detect, by using the contact tracing application, an operation of uploading diagnosis information by the target user (for example, a tap operation of the target user on a "diagnosis information uploading" switch control in a user interface 610 shown in FIG. 6A). In response to the operation, the electronic device 101 may send the diagnosis information to the server 300. In addition, as shown in FIG. 6B, the electronic device 101 may show that the "diagnosis information uploading" switch control is in an on state. After receiving the diagnosis information sent by the electronic device 101, the server 300 may send the diagnosis information to the server 200. The diagnosis information may include information such as a name, a gender, and identification information of the target user.

S517 and S518: The server 200 confirms authenticity of the diagnosis information, and sends confirmation information to the server 300 after the confirmation is completed.

Specifically, after receiving the diagnosis information sent by the server 300, the server 200 may confirm, based on target user information in the public service institution, whether the diagnosis information sent by the server 300 is authentic, to avoid occurrence of a social panic event caused by false reporting of target user information by some users.

If the server 200 confirms that the diagnosis information is authentic, the server 200 may send the confirmation information to the server 300, to prompt the server 300 to perform subsequent steps based on the diagnosis information.

If the server 200 confirms that the diagnosis information is not authentic, the server 200 may also send the confirmation information to the server 300, to prompt the server 300 not to perform subsequent steps.

In a possible implementation, the server 200 may periodically send the target user information to the server 300, and the server 300 does not need to actively send the diagnosis information to the server 200. That is, after receiving the diagnosis information uploaded by the target user, the server 300 may match the diagnosis information against the target user information periodically sent by the server 200. If matching succeeds, step S519 and subsequent steps are performed.

In a possible implementation, the server 300 does not require a user to actively report diagnosis information to the server 300, that is, the server 300 may not need to perform step S515 to step S518. The server 200 may periodically send target user information to the server 300. Target user information sent by the server 200 each time may be user information (for example, information such as a name, a gender, and identification information) of one target user, or may be a user information set of a plurality of target users. After receiving the target user information, the server 300 may perform step S519 and subsequent steps.

S519 and S520: The server 300 searches for a periodic key of the target user, derives a working key based on the periodic key to obtain a working key set, and then sends the working key set of the target user to the electronic device 100.

S521 and S522: The electronic device 100 performs contact prediction and evaluation based on the working key set of the target user, and after the prediction and evaluation are completed, the electronic device 100 displays a contact prediction and evaluation result.

For a specific execution process of step S519 to step S522, refer to related content in step S216 to step S219 in the embodiment shown in FIG. 2A to FIG. 2C. Details are not described herein again.

### Phase 4: contact information uploading and contact chain generation

S523 and S524: The electronic device 100 detects an operation that the suspected target user confirms uploading of contact information. In response to the operation, the electronic device 100 sends the contact information to the server 300.

S525: The server 300 generates a contact chain based on the contact information.

For a specific execution process of step S523 to step S525, refer to related content in step S220 to step S222 in the embodiment shown in FIG. 2A to FIG. 2C. Details are not described herein again.

In some embodiments, the server 300 may generate a contact chain of the user of the electronic device 101->a user of an electronic device 102->a user of an electronic device 103. For a specific process, refer to related text descriptions about FIG. 4A to FIG. 4D. Details are not described herein again.

It should be noted that, in this embodiment of this application, only the application scenario of contact tracing for people related to an infectious disease is used as an example to describe the specific procedure of the contact tracing method in detail. Not limited to the application scenario of contact tracing for people related to an infectious disease, the contact tracing method provided in this embodiment of this application is also applicable to another application scenario (for example, lost property search). This is not limited herein.

In embodiments of this application, a first electronic device may be the electronic device 101, a second electronic device may be the electronic device 100, a third electronic device may be the electronic device 102, a first server may be the server 200, a second server may be the server 300, a short-range wireless communication connection may be the Bluetooth communication connection, a first periodic key may be the periodic key of the electronic device 101, a first working key may be a working key derived by the electronic device 101 based on the first periodic key, a first working key set may include all working keys derived by the electronic device 101 based on the first periodic key, first user information may be the user registration information of the electronic device 101, second user information may be the user registration information of the electronic device 100, third user information may be the user registration information of the electronic device 102, a first derivation algorithm may be the key derivation algorithm, the first contact information may be contact information sent by the electronic device 100 to the server, a second periodic key may be the periodic key of the electronic device 100, a second working key may be a working key derived by the electronic device 100 based on the first periodic key, a second working key set may include all working keys derived by the electronic device 100 based on the second periodic key, first prompt information may be information that is output by the electronic device 100 and that indicates whether a user was in contact with the user of the electronic device 101, a first operation may be an operation that the user of the electronic device 100 confirms uploading of the first contact information, the first contact information may be contact information sent by the electronic device 102 to the server, second prompt information may be information that is output by the electronic device 102 and that indicates whether a user was in contact with the user of the electronic device 100, and a second operation may be an operation that the user of the electronic device 100 confirms uploading of the second contact information.

The following describes a structure of an electronic device 100 provided in an embodiment of this application.

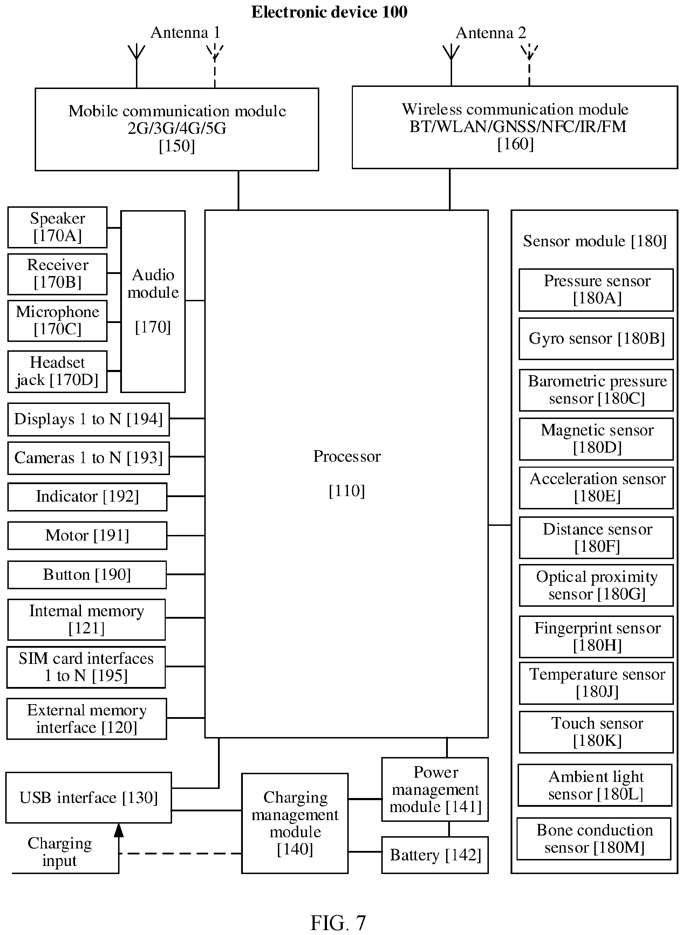

FIG. 7 shows an example of a structure of an electronic device 100 according to an embodiment of this application.

As shown in FIG. 7, the electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present invention does not constitute a specific limitation on the electronic device 100. In some other embodiments of this application, the electronic device 100 may include more or fewer components than those shown in the figure, or combine some components, or split some components, or have different component arrangements. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, and/or a neural-network processing unit (neural-network processing unit, NPU). Different processing units may be independent components, or may be integrated into one or more processors.

The controller may be a nerve center and a command center of the electronic device 100. The controller may generate an operation control signal based on instruction operation code and a time sequence signal, to complete control of instruction fetching and instruction execution.

A memory may be further disposed in the processor 110, to store instructions and data. In some embodiments, the memory in the processor 110 is a cache. The memory may store instructions or data just used or cyclically used by the processor 110. If the processor 110 needs to use the instructions or data again, the instructions or data may be directly invoked from the memory. This avoids repeated access, and reduces waiting time of the processor 110, so that system efficiency is improved.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronous serial bus, and includes a serial data line (serial data line, SDA) and a serial clock line (derail clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K by using an I2C interface, so that the processor 110 communicates with the touch sensor 180K by using the I2C bus interface, to implement a touch function of the electronic device 100.

The I2S interface may be used for audio communication. In some embodiments, the processor 110 may include a plurality of groups of I2S buses. The processor 110 may be coupled to the audio module 170 by using the I2S bus, to implement communication between the processor 110 and the audio module 170. In some embodiments, the audio module 170 may transmit an audio signal to the wireless communication module 160 through the I2S interface, to implement a function of answering a call by using a Bluetooth headset.

The PCM interface may be configured to perform audio communication, and sample, quantize, and code analog signals. In some embodiments, the audio module 170 may be coupled to the wireless communication module 160 by using a PCM bus interface. In some embodiments, the audio module 170 may also transmit an audio signal to the wireless communication module 160 through the PCM interface, to implement a function of answering a call by using a Bluetooth headset. Both the I2S interface and the PCM interface may be used for audio communication.

The UART interface is a universal serial data bus, and is used for asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 by using the UART interface, to implement a Bluetooth function. In some embodiments, the audio module 170 may transmit an audio signal to the wireless communication module 160 through the UART interface, to implement a function of playing music by using a Bluetooth headset.

The MIPI interface may be configured to connect the processor 110 to a peripheral component such as the display 194 or the camera 193. The MIPI interface includes a camera serial interface (camera serial interface, CSI), a display serial interface (display serial interface, DSI), and the like. In some embodiments, the processor 110 and the camera 193 communicate with each other by using the CSI interface, to implement a photographing function of the electronic device 100. The processor 110 communicates with the display 194 by using the DSI interface, to implement a display function of the electronic device 100.

The GPIO interface may be configured by using software. The GPIO interface may be configured as a control signal, or may be configured as a data signal. In some embodiments, the GPIO interface may be configured to connect the processor 110 to the camera 193, the display 194, the wireless communication module 160, the audio module 170, the sensor module 180, and the like. The GPIO interface may alternatively be configured as the I2C interface, the I2S interface, the UART interface, the MIPI interface, or the like.

The USB interface 130 is an interface conforming to a USB standard specification, and may be specifically a mini USB interface, a micro USB interface, a USB Type-C interface, or the like. The USB interface 130 may be configured to connect to a charger to charge the electronic device 100, or may be configured to transmit data between the electronic device 100 and a peripheral device, or may be configured to connect to a headset to play audio by using the headset. The interface may alternatively be configured to connect to another terminal device, such as an AR device.

It may be understood that the interface connection relationship between the modules shown in this embodiment of the present invention is merely an example, and does not constitute a limitation on the structure of the electronic device 100. In some other embodiments of this application, the electronic device 100 may alternatively use an interface connection manner different from that in the foregoing embodiment, or a combination of a plurality of interface connection manners.

The charging management module 140 is configured to receive charging input from a charger. The charger may be a wireless charger, or may be a wired charger. In some wired charging embodiments, the charging management module 140 may receive charging input from a wired charger through the USB interface 130. In some wireless charging embodiments, the charging management module 140 may receive wireless charging input through a wireless charging coil of the electronic device 100. When charging the battery 142, the charging management module 140 may further supply power to the electronic device 100 through the power management module 141.

The power management module 141 is configured to connect the battery 142, the charging management module 140, and the processor 110. The power management module 141 receives input from the battery 142 and/or the charging management module 140, and supplies power to the processor 110, the internal memory 121, the external memory, the display 194, the camera 193, the wireless communication module 160, and the like. The power management module 141 may be further configured to monitor parameters such as a battery capacity, a battery cycle count, and a battery health status (leakage or impedance). In some other embodiments, the power management module 141 may alternatively be disposed in the processor 110. In some other embodiments, the power management module 141 and the charging management module 140 may alternatively be disposed in a same component.

A wireless communication function of the electronic device 100 may be implemented by using the antenna 1, the antenna 2, the mobile communication module 150, the wireless communication module 160, the modem processor, the baseband processor, and the like.

The antenna 1 and the antenna 2 are configured to transmit and receive electromagnetic wave signals. Each antenna in the electronic device 100 may be configured to cover one or more communication bands. Different antennas may be multiplexed to improve antenna utilization. For example, the antenna 1 may be multiplexed into a diversity antenna of a wireless local area network. In some other embodiments, the antenna may be used in combination with a tuning switch.

The mobile communication module 150 may provide a solution for wireless communication, including 2G/3G/4G/5G and the like, that is applied to the electronic device 100. The mobile communication module 150 may include at least one filter, a switch, a power amplifier, a low noise amplifier (low noise amplifier, LNA), and the like. The mobile communication module 150 may receive an electromagnetic wave by using the antenna 1, perform processing such as filtering and amplification on the received electromagnetic wave, and send a processed electromagnetic wave to the modem processor for demodulation. The mobile communication module 150 may further amplify a signal modulated by the modem processor, and convert the signal into an electromagnetic wave for radiation through the antenna 1. In some embodiments, at least some function modules of the mobile communication module 150 may be disposed in the processor 110. In some embodiments, at least some function modules of the mobile communication module 150 may be disposed in a same component as at least some modules of the processor 110.

The modem processor may include a modulator and a demodulator. The modulator is configured to adjust a to-be-sent low-frequency baseband signal to a medium/high-frequency signal. The demodulator is configured to demodulate a received electromagnetic wave signal into a low-frequency baseband signal. Then, the demodulator transmits the low-frequency baseband signal obtained through demodulation to the baseband processor for processing. After being processed by the baseband processor, the low-frequency baseband signal is transmitted to the application processor. The application processor outputs a sound signal by using an audio device (which is not limited to the speaker 170A, the receiver 170B, or the like), or displays an image or a video by using the display 194. In some embodiments, the modem processor may be an independent device. In some other embodiments, the modem processor may be independent of the processor 110 and disposed in a same device as the mobile communication module 150 or another function module.

The wireless communication module 160 may provide a solution for wireless communication that is applied to the electronic device 100 and that includes a wireless local area network (wireless local area networks, WLAN) (such as a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, and the like. The wireless communication module 160 may be one or more components that integrate at least one communication processing module. The wireless communication module 160 receives an electromagnetic wave through the antenna 2, performs frequency modulation and filtering on an electromagnetic wave signal, and sends a processed signal to the processor 110. The wireless communication module 160 may further receive a to-be-sent signal from the processor 110, perform frequency modulation and amplification on the to-be-sent signal, and convert the to-be-sent signal into an electromagnetic wave for radiation through the antenna 2.

In some embodiments, in the electronic device 100, the antenna 1 is coupled to the mobile communication module 150, and the antenna 2 is coupled to the wireless communication module 160, so that the electronic device 100 can communicate with a network and another device according to a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, GNSS, WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation systems, SBAS).

The electronic device 100 implements a display function by using the GPU, the display 194, the application processor, and the like. The GPU is a microprocessor for image processing, and is connected to the display 194 and the application processor. The GPU is configured to perform mathematical and geometric calculation and render graphics. The processor 110 may include one or more GPUs that execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flex light-emitting diode, FLED), a mini-LED, a micro-LED, a micro-OLED, a quantum dot light-emitting diode (quantum dot light-emitting diodes, QLED), or the like. In some embodiments, the electronic device 100 may include one or N displays 194, where N is a positive integer greater than 1.

The electronic device 100 may implement a photographing function by using the ISP, the camera 193, the video codec, the GPU, the display 194, the application processor, and the like.

The ISP is configured to process data fed back by the camera 193. For example, during photographing, a shutter is pressed, light is transmitted to a photosensitive element of the camera through a lens, an optical signal is converted into an electrical signal, and the photosensitive element of the camera transmits the electrical signal to the ISP for processing, to convert the electrical signal into a visible image. The ISP may further perform algorithm optimization on noise, luminance, and complexion of the image. The ISP may further optimize parameters such as exposure and a color temperature of a photographing scenario. In some embodiments, the ISP may be disposed in the camera 193.

The camera 193 is configured to capture a still image or a video. An optical image of an object is generated by using a lens and projected onto a photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP for conversion into a digital image signal. The ISP outputs the digital image signal to the DSP for processing. The DSP converts the digital image signal into a standard image signal in a format such as RGB or YUV. In some embodiments, the electronic device 100 may include one or N cameras 193, where N is a positive integer greater than 1.

The digital signal processor is configured to process a digital signal. In addition to processing a digital image signal, the digital signal processor may further process another digital signal. For example, when the electronic device 100 selects a frequency, the digital signal processor is configured to perform Fourier transform or the like on frequency energy.

The video codec is configured to compress or decompress a digital video. The electronic device 100 may support one or more types of video codecs. In this way, the electronic device 100 may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor that processes input information rapidly by referring to a structure of a biological neural network, for example, by referring to a transmission mode between human brain neurons, and can further perform self-learning continuously. The NPU may be used to implement applications such as intelligent cognition of the electronic device 100, for example, image recognition, facial recognition, voice recognition, and text understanding.

The external memory interface 120 may be configured to connect to an external memory card, such as a micro SD card, to extend a storage capability of the electronic device 100. The external memory card communicates with the processor 110 through the external memory interface 120, to implement a data storage function. For example, files such as music or videos are stored in the external memory card.

The internal memory 121 may be configured to store computer executable program code, where the executable program code includes instructions. The processor 110 performs various function applications and data processing of the electronic device 100 by running the instructions stored in the internal memory 121. The internal memory 121 may include a program storage area and a data storage area. The program storage area may store an operating system, an application program required by at least one function (for example, a sound play function or an image play function), and the like. The data storage area may store data (such as audio data or a phone book) created in a use process of the electronic device 100. In addition, the internal memory 121 may include a high-speed random access memory, and may further include a non-volatile memory, such as at least one magnetic disk storage component, a flash memory component, and a universal flash storage (universal flash storage, UFS).

The electronic device 100 may implement an audio function, such as music playing and recording, by using the audio module 170, the speaker 170A, the receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be further configured to encode and decode audio signals. In some embodiments, the audio module 170 may be disposed in the processor 110, or some function modules of the audio module 170 may be disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an audio electrical signal into a sound signal. When a call is answered or audio information is listened to by using the electronic device 100, the receiver 170B may be put close to a human ear to listen to a voice.

The microphone 170C, also referred to as a "mike" or a "mic", is configured to convert a sound signal into an electrical signal. When making a call or sending a voice message, the user may make a sound near the microphone 170C through the mouth, to enter a sound signal to the microphone 170C. At least one microphone 170C may be disposed on the electronic device 100. In some other embodiments, two microphones 170C may be disposed on the electronic device 100. In addition to sound signal collection, a noise reduction function may be further implemented. In some other embodiments, three, four, or more microphones 170C may be alternatively disposed on the electronic device 100, to collect a sound signal, implement noise reduction, recognize a sound source, implement a directional recording function, and the like.

The headset jack 170D is configured to connect to a wired headset. The headset jack 170D may be the USB interface 130, or may be a 3.5 mm open mobile terminal platform (open mobile terminal platform, OMTP) standard interface or a cellular telecommunications industry association of the USA (cellular telecommunications industry association of the USA, CTIA) standard interface.

The pressure sensor 180A is configured to sense a pressure signal, and may convert the pressure signal into an electrical signal. In some embodiments, the pressure sensor 180A may be disposed on the display 194. There are many types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines strength of the pressure based on the change of the capacitance. When a touch operation is performed on the display 194, the electronic device 100 detects strength of the touch operation through the pressure sensor 180A. The electronic device 100 may also calculate a position of the touch based on a detection signal of the pressure sensor 180A. In some embodiments, touch operations performed on a same touch position but has different touch operation strength may correspond to different operation instructions. For example, when a touch operation whose touch operation strength is less than a first pressure threshold is applied to a Messages icon, an instruction for viewing an SMS message is executed. When a touch operation whose touch operation strength is greater than or equal to the first pressure threshold is applied to the Messages icon, an instruction for creating an SMS message is executed.

The gyro sensor 180B may be configured to determine a motion gesture of the electronic device 100. In some embodiments, angular velocities of the electronic device 100 around the three axes (that is, the x-axis, the y-axis, and the z-axis) may be determined by using the gyro sensor 180B. The gyro sensor 180B may be used for image stabilization during photographing. For example, when a shutter is pressed, the gyro sensor 180B detects a jittering angle of the electronic device 100, calculates, based on the angle, a distance for which a lens module needs to compensate, and enables the lens to offset jittering of the electronic device 100 through reverse motion, so as to implement image stabilization. The gyro sensor 180B may be further used in a navigation scenario and a motion-controlled gaming scenario.

The barometric pressure sensor 180C is configured to measure barometric pressure. In some embodiments, the electronic device 100 calculates an altitude based on a barometric pressure value measured by the barometric pressure sensor 180C, to assist in positioning and navigation.

The magnetic sensor 180D includes a Hall sensor. The electronic device 100 may detect opening and closing of a flip cover by using the magnetic sensor 180D. In some embodiments, when the electronic device 100 is a flip phone, the electronic device 100 may detect opening and closing of a flip cover based on the magnetic sensor 180D. Further, a feature such as automatic unlocking upon opening of the flip cover is set based on a detected opening or closing state of the flip cover.

The acceleration sensor 180E may detect values of acceleration of the electronic device 100 in all directions (usually on three axes). When the electronic device 100 is static, the acceleration sensor 180E may detect magnitude and a direction of gravity. The acceleration sensor 180E may be further configured to recognize a posture of the electronic device 100, and is applied to an application such as switching between landscape mode and vertical mode and a pedometer.

The distance sensor 180F is configured to measure a distance. The electronic device 100 may measure a distance through infrared or laser. In some embodiments, in a photographing scenario, the electronic device 100 may measure a distance by using the distance sensor 180F, to implement quick focusing.

The optical proximity sensor 180G may include, for example, a light-emitting diode (LED) and a light detector such as a photodiode. The light-emitting diode may be an infrared light-emitting diode. The electronic device 100 emits infrared light by using the light-emitting diode. The electronic device 100 detects infrared reflected light from a nearby object by using the photodiode. When sufficient reflected light is detected, the electronic device 100 may determine that there is an object near the electronic device 100. When insufficient reflected light is detected, the electronic device 100 may determine that there is no object near the electronic device 100. The electronic device 100 may detect, by using the optical proximity sensor 180G, that a user holds the electronic device 100 close to the ear for a call, to automatically turn off a screen to save power. The optical proximity sensor 180G may also be used in a flip cover mode or a pocket mode to automatically perform screen unlocking or locking.

The ambient light sensor 180L is configured to sense ambient light brightness. The electronic device 100 may adaptively adjust brightness of the display 194 based on the sensed ambient light brightness. The ambient light sensor 180L may also be configured to automatically adjust a white balance during photographing. The ambient light sensor 180L may further cooperate with the optical proximity sensor 180G to detect whether the electronic device 100 is in a pocket, to prevent an accidental touch.

The fingerprint sensor 180H is configured to collect a fingerprint. The electronic device 100 may implement fingerprint-based unlocking, application lock access, fingerprint-based photographing, fingerprint-based call answering, or the like based on a feature of the collected fingerprint.

The temperature sensor 180J is configured to detect a temperature. In some embodiments, the electronic device 100 executes a temperature processing policy based on the temperature detected by the temperature sensor 180J. For example, when the temperature reported by the temperature sensor 180J exceeds a threshold, the electronic device 100 reduces performance of a processor near the temperature sensor 180J, to reduce power consumption and implement thermal protection. In some other embodiments, when the temperature is lower than another threshold, the electronic device 100 heats the battery 142 to avoid abnormal shutdown of the electronic device 100 that is caused due to a low temperature. In some other embodiments, when the temperature is lower than still another threshold, the electronic device 100 boosts an output voltage of the battery 142, to avoid an abnormal shutdown caused by a low temperature.

The touch sensor 180K is also referred to as a "touch panel". The touch sensor 180K may be disposed on the display 194. The touch sensor 180K and the display 194 form a touchscreen, which is also referred to as a "touch screen". The touch sensor 180K is configured to detect a touch operation performed on or near the touch sensor. The touch sensor may transfer the detected touch operation to the application processor to determine a type of a touch event. Visual output related to the touch operation may be provided by using the display 194. In some other embodiments, the touch sensor 180K may alternatively be disposed on a surface of the electronic device 100 at a position different from that of the display 194.

The bone conduction sensor 180M may obtain a vibration signal. In some embodiments, the bone conduction sensor 180M may obtain a vibration signal of a vibration bone of a human vocal cord part. The bone conduction sensor 180M may also be in contact with a human pulse, and receive a blood pressure beating signal. In some embodiments, the bone conduction sensor 180M may alternatively be disposed in the headset, to form a bone conduction headset. The audio module 170 may obtain a voice signal through parsing based on the vibration signal, of the vibration bone of the vocal cord part, obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive button input, and generate button signal input related to user settings and function control of the electronic device 100.

The motor 191 may generate a vibration prompt. The motor 191 may be used for an incoming call vibration prompt, or may be used for touch vibration feedback. For example, touch operations performed on different applications (for example, photo taking and audio playing) may be corresponding to different vibration feedback effects. The motor 191 may also correspond to different vibration feedback effects for touch operations performed in different regions of the display 194. Different application scenarios (for example, a time reminder, information receiving, an alarm clock, and a game) may also be corresponding to different vibration feedback effects. A touch vibration feedback effect may be further customized.

The indicator 192 may be an indicator light, and may indicate a charging status or a power change, or may indicate a message, a missed call, a notification, or the like.

The SIM card interface 195 is configured to connect to a SIM card. The SIM card may be inserted into the SIM card interface 195 or pulled out of the SIM card interface 195, to implement contact with or separation from the electronic device 100. The electronic device 100 may support one or N SIM card interfaces, and N is a positive integer greater than 1. The SIM card interface 195 can support a nano-SIM card, a micro-SIM card, a SIM card, and the like. A plurality of cards may be simultaneously inserted into a same SIM card interface 195. The plurality of cards may be of a same type or different types. The SIM card interface 195 may also be compatible with different types of SIM cards. The SIM card interface 195 may also be compatible with an external memory card. The electronic device 100 interacts with a network by using a SIM card, to implement functions such as conversation and data communication. In some embodiments, the electronic device 100 uses an eSIM, namely, an embedded SIM card. The eSIM card may be embedded in the electronic device 100, and cannot be separated from the electronic device 100.

It should be understood that the electronic device 100 shown in FIG. 7 is only an example, and the electronic device 100 may have more or fewer components than those shown in FIG. 7, may combine two or more components, or may have different component configurations. The components shown in FIG. 7 may be implemented in hardware including one or more signal processing and/or application-specific integrated circuits, software, or a combination of hardware and software.

The following describes a software structure of an electronic device 100 provided in an embodiment of this application.

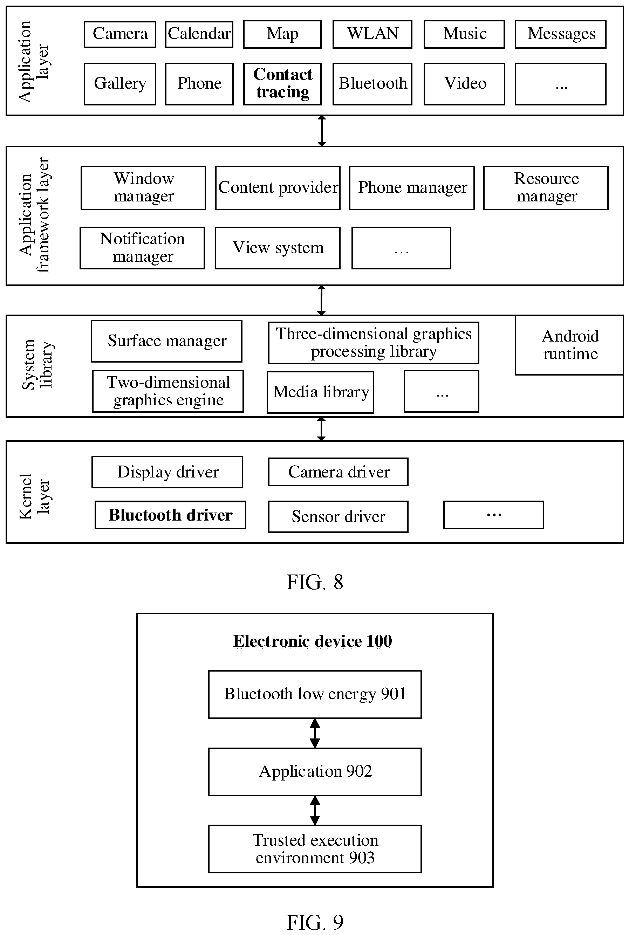

FIG. 8 shows an example of a software structure of an electronic device 100 according to an embodiment of this application.

As shown in FIG. 8, a software system of the electronic device 100 may use a layered architecture, an event-driven architecture, a microkernel architecture, a micro-service architecture, or a cloud architecture. In this embodiment of this application, an Android system with a layered architecture is used as an example to describe the software structure of the electronic device 100.

In the layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom.

The application layer may include a series of application packages.

As shown in FIG. 8, the application packages may include applications such as Camera, Gallery, Calendar, Phone, Map, Contact Tracing, WLAN, Bluetooth, Music, Video, and Messages.

The application framework layer provides an application programming interface (application programming interface, API) and a programming framework for applications at the application layer. The application framework layer includes some predefined functions.

As shown in FIG. 8, the application framework layer may include a window manager, a content provider, a view system, a phone manager, a resource manager, a notification manager, and the like.

The window manager is configured to manage window programs. The window manager may obtain a display size, determine whether there is a status bar, lock a screen, take a screenshot, and the like.

The content provider is configured to store and obtain data and make the data accessible to the application. The data may include videos, images, audio, calls that are made and received, browsing history and bookmarks, a phone book, and the like.

The view system includes visual controls, such as a text display control and a picture display control. The view system may be configured to construct an application. A display interface may include one or more views. For example, a display interface including an SMS notification icon may include a text display view and a picture display view.

The phone manager is configured to provide a communication function of the electronic device 100, for example, call status management (including accepting and declining).

The resource manager provides various resources for the application, such as a localized string, an icon, a picture, a layout file, and a video file.

The notification manager enables the application to display notification information in a status bar, and may be configured to transfer a message of a notification type. The information may automatically disappear after a short stay without user interaction. For example, the notification manager is configured to notify a download completion, a message reminder, and the like. The notification manager may alternatively be a notification that appears in a form of a graph or a scroll bar text in a status bar at the top of the system, for example, a notification of an application running in the background, or may be a notification that appears in a form of a dialog window on a screen. For example, the status bar shows text information, a prompt tone is made, the electronic device vibrates, and an indicator flickers.

The Android runtime includes a kernel library and a virtual machine. The Android runtime is responsible for scheduling and management of the Android system.

The kernel library includes two parts: a function that needs to be invoked by using the Java language, and a kernel library of Android.

The application layer and the application framework layer run in the virtual machine. The virtual machine executes a binary file of a Java file at the application layer and the application framework layer. The virtual machine is configured to perform functions such as object lifecycle management, stack management, thread management, security and abnormity management, and garbage collection.

The system library may include a plurality of function modules, for example, a surface manager (surface manager), a media library (Media Libraries), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in a plurality of commonly used audio and video formats, and static image files. The media library may support a plurality of audio and video coding formats, such as MPEG-4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a camera driver, a Bluetooth driver, and a sensor driver.

The following describes an example of a working process of software and hardware of the electronic device 100 with reference to a photographing scenario.

When the touch sensor 180K receives a touch operation, a corresponding hardware interrupt is sent to the kernel layer. The kernel layer processes the touch operation into an original input event (including information such as touch coordinates and a timestamp of the touch operation). The original input event is stored at the kernel layer. The application framework layer obtains the original input event from the kernel layer, and identifies a control corresponding to the input event. For example, the touch operation is a single-tap touch operation, and a control corresponding to the single-tap operation is a control of a camera application icon. The camera application invokes an interface of the application framework layer to enable a camera application, then enables a camera driver by invoking the kernel layer, and captures a static image or a video through the camera 193.

The following describes a structure of another electronic device 100 provided in an embodiment of this application.

FIG. 9 shows an example of a structure of another electronic device 100 according to an embodiment of this application.

As shown in FIG. 9, the electronic device 100 may include Bluetooth low energy 901, an application 902, and a trusted execution environment 903.

The Bluetooth low energy 901 may be configured to send and receive Bluetooth low energy advertising packets between different electronic devices. For example, the electronic device 100 may generate a Bluetooth low energy advertising packet based on a working key, and then periodically send the Bluetooth low energy advertising packet to another surrounding electronic device through the Bluetooth low energy 901. For another example, the electronic device 100 may further periodically scan, through the Bluetooth low energy 901, a Bluetooth low energy advertising packet sent by another surrounding electronic device, and receive the Bluetooth low energy advertising packet.

The application 902 is the contact tracing application described above, and may be configured to generate a periodic key and a working key of a user, predict, evaluate, and display a contact result, report contact information, and the like. For example, after the user completes registration and login operations, the application 902 may generate a periodic key, and derive a working key based on the periodic key. For another example, the application 902 may perform contact prediction and evaluation based on a working key set of a target user, and display a contact prediction and evaluation result after the contact prediction and evaluation are completed. For another example, the application 902 may send contact information of a suspected target user to a server.

The trusted execution environment 903 may be configured to encrypt and store a periodic key generated in the application 902.

For more details about a function and a working principle of the electronic device 100, refer to related content in the foregoing embodiments. Details are not described herein again.

The following describes a structure of a server 200 provided in an embodiment of this application.

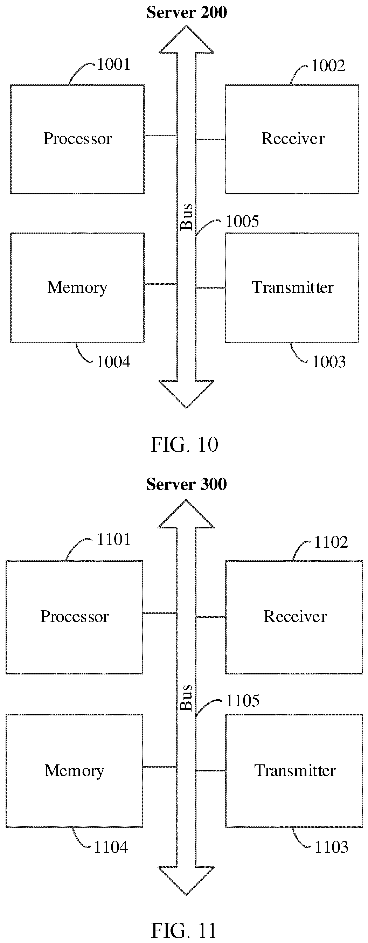

FIG. 10 shows an example of a structure of a server 200 according to an embodiment of this application.

As shown in FIG. 10, the server 200 may include a processor 1001, a receiver 1002, a transmitter 1003, a memory 1004, and a bus 1005. The processor 1001, the receiver 1002, the transmitter 1003, and the memory 1004 are connected to each other through the bus 1005. The processor 1001 includes one or more processing cores, and the processor 1001 applies various functions and processes information by running a software program and a module. The receiver 1002 and the transmitter 1003 may be implemented as one communication component, and the communication component may be a baseband chip. The memory 1004 may be configured to store at least one program instruction, and the processor 1001 is configured to execute the at least one program instruction, to implement the technical solutions in the foregoing embodiments.

The processor 1001 may be configured to run the at least one program instruction stored in the memory 1004, to perform the following operations:
searching for a periodic key of a target user, and deriving a working key based on the periodic key;
confirming authenticity of received diagnosis information; and
generating a contact chain based on contact information of a suspected target user.

The receiver 1002 may be configured to receive information sent by an electronic device 100 and a server 300. For example, the receiver 1002 may receive user registration information, a periodic key, and contact information that are sent by the electronic device 100. For another example, the receiver 1002 may receive diagnosis information sent by the server 300.

The transmitter 1003 may be configured to send information to the electronic device 100 and the server 300. For example, the transmitter 1003 may send a working key set of a target user to the electronic device 100. For another example, the transmitter 1003 may send confirmation information for confirming authenticity of the diagnosis information to the server 300.

The memory 1004 may be configured to store information sent by the electronic device 100. For example, the memory 1004 may store the user registration information, the periodic key, and the contact information that are sent by the electronic device 100.

For more details about a function and a working principle of the server 200, refer to related content in the foregoing embodiments. Details are not described herein again.

The following describes a structure of another server 300 provided in an embodiment of this application.

FIG. 11 shows an example of a structure of another server 300 according to an embodiment of this application.

As shown in FIG. 11, the server 300 may include a processor 1101, a receiver 1102, a transmitter 1103, a memory 1104, and a bus 1105. The processor 1101, the receiver 1102, the transmitter 1103, and the memory 1104 are connected to each other through the bus 1105. The processor 1101 includes one or more processing cores, and the processor 1101 applies various functions and processes information by running a software program and a module. The receiver 1102 and the transmitter 1103 may be implemented as one communication component, and the communication component may be a baseband chip. The memory 1104 may be configured to store at least one program instruction, and the processor 1101 is configured to execute the at least one program instruction, to implement the technical solutions in the foregoing embodiments.

The processor 1101 may be configured to run the at least one program instruction stored in the memory 1104, to perform the following operations:
searching for a periodic key of a target user, and deriving a working key based on the periodic key; and
generating a contact chain based on contact information of a suspected target user.

The receiver 1102 may be configured to receive information sent by an electronic device 100 and a server 200. For example, the receiver 1102 may receive user registration information, a periodic key, and contact information that are sent by the electronic device 100. For another example, the receiver 1102 may receive confirmation information that is for confirming authenticity of diagnosis information and that is sent by the server 200.

The transmitter 1103 may be configured to send information to the electronic device 100 and the server 200. For example, the transmitter 1103 may send a working key set of a target user to the electronic device 100. For another example, the transmitter 1103 may send the diagnosis information to the server 200.

The memory 1104 may be configured to store information sent by the electronic device 100. For example, the memory 1104 may store the user registration information, the periodic key, and the contact information that are sent by the electronic device 100.

For more details about a function and a working principle of the server 300, refer to related content in the foregoing embodiments. Details are not described herein again.

The foregoing embodiments are merely intended for describing the technical solutions of this application, but not for limiting this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of embodiments of this application.

## Claims

1. A contact tracing method, applied to a communication system comprising a first electronic device, a second electronic device, and a first server, wherein the method comprises:
establishing, by the first electronic device, a short-range wireless communication connection to the second electronic device;
generating, by the first electronic device, a first periodic key, and sending the first periodic key to the first server;
sending, by the first electronic device, a first working key to the second electronic device through the short-range wireless communication connection, wherein the first working key is derived by the first electronic device based on the first periodic key;
receiving and storing, by the second electronic device, the first working key;
determining, by the first server, that a user of the first electronic device is a target user;
deriving, by the first server, a first working key set based on the first periodic key, and sending the first working key set to the second electronic device; and
if the second electronic device obtains the first working key set and determines that the first working key set comprises the first working key, determining, by the second electronic device, that a user of the second electronic device is a suspected target user.

2. The method according to claim 1, wherein the first periodic key is periodically generated by the first electronic device and sent to the first server.

3. The method according to claim 1 or 2, wherein before the sending, by the first electronic device, a first working key to the second electronic device through the short-range wireless communication connection, the method further comprises:
sending, by the first electronic device, first user information to the first server, wherein the first user information is associated with the first periodic key; and
sending, by the second electronic device, second user information to the first server.

4. The method according to claims 1 to 3, wherein after the establishing, by the first electronic device, a short-range wireless communication connection to the second electronic device, the method further comprises:
sending, by the second electronic device, a second working key to the first electronic device, wherein the second working key is derived by the second electronic device based on a second periodic key by using a first derivation algorithm, the second periodic key is associated with the second user information, and the second periodic key is periodically generated by the second electronic device and sent to the first server; and
receiving and storing, by the first electronic device, the second working key.

5. The method according to claim 4, wherein the first working key is derived by the first electronic device from the first periodic key by using the first derivation algorithm, and the first working key set is derived by the first server from the first periodic key by using the first derivation algorithm.

6. The method according to any one of claims 1 to 5, wherein after the determining, by the second electronic device, that a user of the second electronic device is a suspected target user, the method further comprises:
sending, by the second electronic device, first contact information and the second user information to the first server, wherein the first contact information comprises the first working key; and
generating, by the first server, a first contact chain based on the first contact information and the second user information, wherein the first contact chain indicates that the user of the first electronic device was in contact with the user of the second electronic device.

7. The method according to claim 6, wherein before the sending, by the second electronic device, first contact information and the second user information to the first server, the method further comprises:
outputting, by the second electronic device, first prompt information, wherein the first prompt information indicates that the user of the second electronic device is the suspected target user; and
detecting, by the second electronic device, a first operation of the user.

8. The method according to claim 6 or 7, wherein the communication system further comprises a third electronic device, and before the generating, by the first server, a first contact chain based on the first contact information and the second user information, the method further comprises:
sending, by the third electronic device, third user information to the first server, wherein the third user information is associated with a third periodic key, and the third periodic key is periodically generated by the third electronic device and sent to the first server;
establishing, by the third electronic device, a short-range wireless communication connection to the second electronic device;
sending, by the third electronic device, a third working key to the second electronic device through the short-range wireless communication connection, wherein the third working key is derived by the third electronic device based on the third periodic key;
receiving and storing, by the second electronic device, the third working key;
sending, by the second electronic device, the second working key to the third electronic device through the short-range wireless communication connection; and
receiving and storing, by the third electronic device, the second working key.

9. The method according to claim 8, wherein after the generating, by the first server, a first contact chain based on the first contact information and the second user information, the method further comprises:
obtaining, by the first server, the second periodic key based on the second user information;
deriving, by the first server, a second working key set based on the second periodic key, and sending the second working key set to the third electronic device; and
if the third electronic device obtains the second working key set and determines that the second working key set comprises the second working key, determining, by the third electronic device, that a user of the third electronic device is a contact user of the suspected target user.

10. The method according to claim 9, wherein after the determining, by the third electronic device, that a user of the third electronic device is a contact user of the suspected target user, the method further comprises:
sending, by the third electronic device, second contact information and the third user information to the first server, wherein the second contact information comprises the second working key; and
generating, by the first server, a second contact chain based on the second contact information and the third user information, wherein the second contact chain indicates that the user of the third electronic device was in contact with the user of the second electronic device.

11. The method according to claim 10, wherein before the sending, by the third electronic device, second contact information and the third user information to the first server, the method further comprises:
outputting, by the third electronic device, second prompt information, wherein the second prompt information indicates that the user of the third electronic device is the contact user of the suspected target user; and
detecting, by the third electronic device, a second operation of the user.

12. A contact tracing method, applied to a communication system comprising a first electronic device, a second electronic device, a first server, and a second server, wherein the method comprises:
establishing, by the first electronic device, a short-range wireless communication connection to the second electronic device;
generating, by the first electronic device, a first periodic key, and sending the first periodic key to the second server;
sending, by the first electronic device, a first working key to the second electronic device through the short-range wireless communication connection, wherein the first working key is derived by the first electronic device based on the first periodic key;
receiving and storing, by the second electronic device, the first working key;
sending, by the first server, first information to the second server;
determining, by the second server based on the first information, that a user of the first electronic device is a target user;
deriving, by the second server, a first working key set based on the first periodic key, and sending the first working key set to the second electronic device; and
if the second electronic device obtains the first working key set and determines that the first working key set comprises the first working key, determining, by the second electronic device, that a user of the second electronic device is a suspected target user.

13. The method according to claim 12, wherein the first information is a set of target user information.

14. The method according to claim 12 or 13, wherein the first information is confirmation information, the confirmation information indicates that the user of the first electronic device is the target user, and before the sending, by the first server, first information to the second server, the method further comprises:
sending, by the first electronic device, diagnosis information to the second server;
sending, by the second server, the diagnosis information to the first server; and
generating, by the first server, the confirmation information based on the diagnosis information.

15. The method according to any one of claims 12 to 14, wherein before the sending, by the first electronic device, a first working key to the second electronic device through the short-range wireless communication connection, the method further comprises:
sending, by the first electronic device, first user information to the second server, wherein the first user information is associated with the first periodic key; and
sending, by the second electronic device, second user information to the second server.

16. The method according to any one of claims 12 to 15, wherein after the determining, by the second electronic device, that a user of the second electronic device is a suspected target user, the method further comprises:
sending, by the second electronic device, first contact information and the second user information to the second server, wherein the first contact information comprises the first working key; and
generating, by the second server, a first contact chain based on the first contact information and the second user information, wherein the first contact chain indicates that the user of the first electronic device was in contact with the user of the second electronic device.

17. The method according to claim 16, wherein before the sending, by the second electronic device, first contact information and the second user information to the second server, the method further comprises:
outputting, by the second electronic device, first prompt information, wherein the first prompt information indicates that the user of the second electronic device is the suspected target user; and
detecting, by the second electronic device, a first operation of the user.

18. A contact tracing method, applied to a second electronic device, wherein the method comprises:
establishing, by the second electronic device, a short-range wireless communication connection to a first electronic device;
receiving, by the second electronic device through the short-range wireless communication connection, a first working key sent by the first electronic device, wherein the first working key is derived by the first electronic device based on a first periodic key, and the first periodic key is generated by the first electronic device and sent to a first server;
receiving and storing, by the second electronic device, the first working key;
receiving, by the second electronic device, a first working key set, wherein the first working key set is sent by the first server to the second electronic device after the first server determines that a user of the first electronic device is a target user, and the first working key set is derived by the first server based on the first periodic key; and
if the second electronic device determines that the first working key set comprises the first working key, determining, by the second electronic device, that a user of the second electronic device is a suspected target user.

19. The method according to claim 18, wherein the first periodic key is periodically generated by the first electronic device and sent to the first server.

20. The method according to claim 18 or 19, wherein the first periodic key is associated with first user information, and the first user information is sent by the first electronic device to the first server before the second electronic device receives, through the short-range wireless communication connection, the first working key sent by the first electronic device.

21. The method according to any one of claims 18 to 20, wherein before the receiving, by the second electronic device through the short-range wireless communication connection, a first working key sent by the first electronic device, the method further comprises:
sending, by the second electronic device, second user information to the first server.

22. The method according to any one of claims 18 to 21, wherein after the establishing, by the second electronic device, a short-range wireless communication connection to a first electronic device, the method further comprises:
sending, by the second electronic device, a second working key to the first electronic device through the short-range wireless communication connection, wherein the second working key is derived by the second electronic device based on a second periodic key by using a first derivation algorithm, the second periodic key is associated with the second user information, and the second periodic key is periodically generated by the second electronic device and sent to the first server.

23. The method according to claim 22, wherein the first working key is derived by the first electronic device from the first periodic key by using the first derivation algorithm, and the first working key set is derived by the first server from the first periodic key by using the first derivation algorithm.

24. The method according to any one of claims 18 to 23, wherein after the determining, by the second electronic device, that a user of the second electronic device is a suspected target user, the method further comprises:
sending, by the second electronic device, first contact information and the second user information to the first server, wherein the first contact information comprises the first working key, the first contact information and the second user information are to be used by the first server to generate a first contact chain, and the first contact chain indicates that the user of the first electronic device was in contact with the user of the second electronic device.

25. The method according to claim 24, wherein before the sending, by the second electronic device, first contact information and the second user information to the first server, the method further comprises:
outputting, by the second electronic device, first prompt information, wherein the first prompt information indicates that the user of the second electronic device is the suspected target user; and
detecting, by the second electronic device, a first operation of the user.

26. The method according to claim 24 or 25, wherein before the sending, by the second electronic device, first contact information and the second user information to the first server, the method further comprises:
establishing, by the second electronic device, a short-range wireless communication connection to a third electronic device;
receiving, by the second electronic device through the short-range wireless communication connection, a third working key sent by the third electronic device, wherein the third working key is derived by the third electronic device based on a third periodic key, the third periodic key is periodically generated by the third electronic device and sent to the first server, the third periodic key is associated with third user information, and the third user information is sent by the third electronic device to the first server before the second electronic device receives, through the short-range wireless communication connection, the third working key sent by the third electronic device; and
sending, by the second electronic device, the second working key to the third electronic device through the short-range wireless communication connection.

27. An electronic device, wherein the electronic device comprises one or more processors and one or more memories, the one or more memories are coupled to the one or more processors, the one or more memories are configured to store computer program code, the computer program code comprises computer instructions, and when the one or more processors execute the computer instructions, the electronic device is enabled to perform the method according to any one of claims 18 to 26.

28. A computer storage medium, wherein the computer storage medium stores a computer program, the computer program comprises program instructions, and when the program instructions are run on an electronic device, the electronic device is enabled to perform the method according to any one of claims 18 to 26.
